(19) Europäisches Patentamt
European Patent Office
Office européen des brevets

(11) EP 4 563 707 A1

(12) EUROPEAN PATENT APPLICATION
published in accordance with Art. 153(4) EPC

(43) Date of publication:
**04.06.2025 Bulletin 2025/23**

(21) Application number: **23846160.2**

(22) Date of filing: **05.07.2023**

(51) International Patent Classification (IPC):
***C12Q 1/02*** *(2006.01)* ***C12M 1/00*** *(2006.01)*
***C12M 1/34*** *(2006.01)* ***C12N 1/12*** *(2006.01)*

(52) Cooperative Patent Classification (CPC):
**C12M 1/00; C12M 1/34; C12N 1/12; C12Q 1/02**

(86) International application number:
**PCT/JP2023/024903**

(87) International publication number:
**WO 2024/024427 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.07.2022 JP 2022118748**

(71) Applicant: **Panasonic Intellectual Property Management Co., Ltd.**
**Kadoma-shi, Osaka 571-0057 (JP)**

(72) Inventor: **KOJIMA, Seiji**
**Kadoma-shi, Osaka 571-0057 (JP)**

(74) Representative: **Grünecker Patent- und Rechtsanwälte**
**PartG mbB**
**Leopoldstraße 4**
**80802 München (DE)**

Remarks:
The complete document including Reference Table(s) and the Sequence Listing(s) can be downloaded from the EPO website

# (54) METHOD FOR DETERMINING OUTER MEMBRANE DETACHMENT IN CYANOBACTERIUM, DEVICE FOR DETERMINING OUTER MEMBRANE DETACHMENT IN CYANOBACTERIUM, AND PROGRAM

(57) The present disclosure provides a method for determining deprivation of an outer membrane of a cyanobacterium with which whether an outer membrane is deprived from a cell wall can be readily determined. The method for determining deprivation of an outer membrane of a cyanobacterium according to the present disclosure includes a measurement step (S01) in which a concentration of at least one amino acid selected from the group consisting of isoleucine, leucine, tyrosine, and phenylalanine in a culture supernatant of a cyanobacterium is measured and a determination step (S02) in which whether an outer membrane of the cyanobacterium is deprived from a cell wall of the cyanobacterium is determined on the basis of the concentration of the at least one amino acid, the concentration being measured in the measurement step.

FIG. 2A

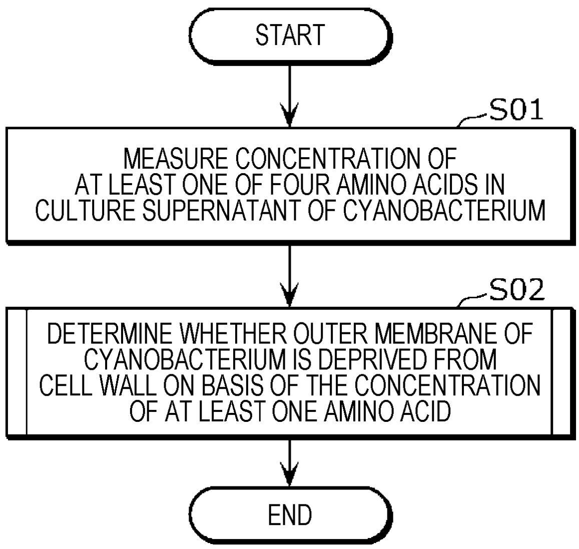


EP 4 563 707 A1

## Description

### Technical Field

The present disclosure relates to a method for determining deprivation of the outer membrane of a cyanobacterium, an apparatus for determining deprivation of the outer membrane of a cyanobacterium, and a program.

### Background Art

Attention has been focused on photosynthetic microorganisms, such as cyanobacteria and algae, as a tool for achieving an environmentally-friendly, next-generation matter production system. Matter production using photosynthetic microorganisms is performed under a normal-temperature, normal-pressure condition using light as an energy source, water, and carbon dioxide ($CO_2$) included in the atmosphere. Since recent progress in the gene manipulation technology enabled us to produce a wide variety of types of compounds using gene recombinant photosynthetic microorganisms, matter production using photosynthetic microorganisms is considered as a promising next-generation technology with which carbon neutrality may be achieved.

As an example of the matter production using photosynthetic microorganisms, a technology of enhancing the matter productivity of a genetically modified strain prepared by modifying a gene of a photosynthetic microorganism which is responsible for metabolism or matter biosynthesis is disclosed. This technology enhances, for example, the productivity of sucrose (NPL 1), isobutanol (NPL 2), fatty acid (NPL 3), amino acid (NPL 4), and protein (NPL 5). Moreover, for example, a technology of enhancing protein productivity of a genetically modified strain prepared by depriving the outer membrane of a cyanobacterium from the cell wall is also disclosed (PTL 1).

### Citation List

#### Patent Literature

PTL 1: International Publication No. 2021/100640

#### Non Patent Literature

NPL 1: Daniel C Ducat et al., "Rerouting Carbon Flux To Enhance Photosynthetic Productivity", Applied and Environmental Microbiology, American Society for Microbiology, April 2012, Vol. 78, No. 8, pp. 2660-2668

NPL 2: Shota Atsumi et al., "Direct photosynthetic recycling of carbon dioxide to isobutyraldehyde", Nature Biotechnology, Nature Publishing Group, November 2009, Vol. 27, No. 12, pp. 1177-1180

NPL 3: Xinyao Liu et al., "Fatty acid production in genetically modified cyanobacteria", The Proceedings of the Natural Academy of Sciences (PNAS), National Academy of Sciences, April 2011, Vol. 108, No. 17, pp. 6899-6904

NPL 4: Arnav Deshpande et al., "Combining random mutagenesis and metabolic engineering for enhanced tryptophan production in Synechocystis sp. strain PCC 6803", Applied Environmental Microbiology, May 2020, Vol. 86, No. 9, e02816-19

NPL 5: James A. Gregory et al., "Alga-Produced Cholera Toxin-Pfs25 Fusion Proteins as Oral Vaccines", Applied and Environmental Microbiology, American Society for Microbiology, June 2013, Vol. 79, No. 13, pp. 3917-3925


### Summary of Invention

#### Technical Problem

However, in the above related art, it is necessary to determine whether the conditions of the cells of the genetically modified strain of photosynthetic microorganisms are suitable for efficient matter production by, for example, inspecting the cells with an electron microscope or using a complex biochemical analytical method, which requires a lot of effort and time.

Accordingly, the present disclosure provides a method for determining deprivation of the outer membrane of a cyanobacterium, an apparatus for determining deprivation of the outer membrane of a cyanobacterium, and a program with which whether the outer membrane is deprived from the cell wall can be readily determined in order to determine whether the conditions of the cells of the cyanobacterium are suitable for efficient matter production.

Solution to Problem

A method for determining deprivation of an outer membrane of a cyanobacterium according to an aspect of the present disclosure includes a measurement step in which a concentration of at least one amino acid selected from the group consisting of isoleucine, leucine, tyrosine, and phenylalanine in a culture supernatant of a cyanobacterium is measured, and a determination step in which whether an outer membrane of the cyanobacterium is deprived from a cell wall of the cyanobacterium is determined on the basis of the concentration of the at least one amino acid, the concentration being measured in the measurement step.

An apparatus for determining deprivation of an outer membrane of a cyanobacterium according to an aspect of the present disclosure include a measurement unit that measures a concentration of at least one amino acid selected from the group consisting of isoleucine, leucine, tyrosine, and phenylalanine in a culture supernatant of a cyanobacterium, and a determination unit that determines whether an outer membrane of the cyanobacterium is deprived from a cell wall of the cyanobacterium on the basis of the concentration of the at least one amino acid, the concentration being measured by the measurement unit.

A program according to an aspect of the present disclosure is a program for causing a computer to execute a method for determining whether an outer membrane of a cyanobacterium is deprived from a cell wall of the cyanobacterium on the basis of a concentration of at least one amino acid selected from the group consisting of isoleucine, leucine, tyrosine, and phenylalanine in a culture supernatant of the cyanobacterium. Advantageous Effects of Invention

With the method for determining deprivation of an outer membrane of a cyanobacterium, the apparatus for determining deprivation of an outer membrane of a cyanobacterium, and the program according to the present disclosure, whether the outer membrane of a cyanobacterium is deprived from the cell wall can be readily determined.

Brief Description of Drawings

[Fig. 1] Fig. 1 is a block diagram illustrating an example of the functional structure of an apparatus for determining deprivation of the outer membrane of a cyanobacterium according to an embodiment.
[Fig. 2A] Fig. 2A is a flowchart illustrating an example of the flow of a method for determining deprivation of the outer membrane of a cyanobacterium according to an embodiment.
[Fig. 2B] Fig. 2B is a flowchart illustrating the detailed flow of Step S02 illustrated in Fig. 2A.
[Fig. 3] Fig. 3 is a diagram schematically illustrating the surface layer of a cyanobacterial cell.
[Fig. 4] Fig. 4 is a transmission electron microscope image of an ultrathin slice of a modified cyanobacterium prepared in Example 1.
[Fig. 5] Fig. 5 includes magnified views of the broken-line region A in Fig. 4.
[Fig. 6] Fig. 6 is a transmission electron microscope image of an ultrathin slice of a modified cyanobacterium prepared in Example 2.
[Fig. 7] Fig. 7 includes magnified views of the broken-line region B in Fig. 6.
[Fig. 8] Fig. 8 is a transmission electron microscope image of an ultrathin slice of a modified cyanobacterium prepared in Comparative Example 1.
[Fig. 9] Fig. 9 includes magnified views of the broken-line region C in Fig. 8.
[Fig. 10] Fig. 10 includes graphs illustrating the mass of proteins in the culture supernatants of the modified cyanobacteria prepared in Example 1, Example 2, and Comparative Example 1 (n = 3, error bar: SD).
[Fig. 11] Fig. 11 is a diagram illustrating the results of amino acid analysis of the culture supernatants of two types of cyanobacteria by LC-MS/MS.
[Fig. 12] Fig. 12 illustrates SEQ ID NOs: 1 to 3.
[Fig. 13] Fig. 13 illustrates SEQ ID NOs: 4 to 6.
[Fig. 14] Fig. 14 illustrates SEQ ID NO: 7.
[Fig. 15] Fig. 15 illustrates SEQ ID NO: 8.
[Fig. 16] Fig. 16 illustrates SEQ ID NO: 9.
[Fig. 17] Fig. 17 illustrates SEQ ID NO: 10.
[Fig. 18] Fig. 18 illustrates SEQ ID NO: 11.
[Fig. 19] Fig. 19 illustrates SEQ ID NOs: 12 to 18.
[Fig. 20] Fig. 20 illustrates SEQ ID NOs: 19 to 22.

Description of Embodiments

(Underlying Knowledge Forming Basis of the Present Disclosure)

Attention has been focused on photosynthetic microorganisms, such as cyanobacteria and algae, as a tool for achieving an environmentally-friendly, next-generation matter production system. In particular, a method for evaluating and managing cyanobacterial cells and the cultivation conditions in an easy and efficient manner is anticipated for the matter production using cyanobacteria.

Under the above circumstances, the present inventor focused attention on cyanobacteria as photosynthetic microorganisms used for matter production. Cyanobacteria (referred to also as "blue-green bacteria" or "blue-green algae") are a group of eubacteria. Cyanobacteria split water by photosynthesis to produce oxygen and fix the $CO_2$ included in the atmosphere using the resulting energy. Some types of cyanobacteria fix nitrogen ($N_2$) included in the atmosphere. One of the known characteristics of cyanobacteria is that they grow fast and have a high light use efficiency. In addition, gene manipulation of cyanobacteria can be done readily compared with other type of algae. Thus, among photosynthetic microorganisms, the use of cyanobacteria has been actively researched and developed. As described above, there have been reports of production of sucrose (NPL 1), isobutanol (NPL 2), fatty acid (NPL 3), amino acid (NPL 4), protein (NPL 5), and the like as examples of matter production using cyanobacteria (specifically, modified cyanobacteria).

For example, NPL 1 discloses that a genetically modified strain prepared by modifying a gene of Synechococcus elongatus which is responsible for the sucrose biosynthesis channel has a higher sucrose productivity than a wild-type strain.

For example, NPL 2 discloses that a genetically modified strain with overexpressed ribulose-1,5-bisphosphate carboxylase/oxidase (Rubisco) which is prepared by manipulating a gene of Synechococcus elongatus PCC7942 has a high isobutanol productivity than a wild-type strain.

For example, NPL 3 discloses that a genetically modified strain prepared by introducing an acyl-acyl transport protein thioesterase gene to Synechocystis sp. PCC6803 has a high fatty acid productivity than a wild-type strain.

NPL 4 discloses that a tryptophan overproducing strain isolated by subjecting a wild-type strain of Synechocystis sp. PCC 6803 strain to random mutagenesis and a selection using amino acids has a high tryptophan productivity than a wild-type strain.

NPL 5 discloses that a genetically modified alga prepared by manipulating the genes of the chloroplast of Chlamydomonas reinhardtii, which is a type of algae, such that a chimeric protein (CtxB-Pfs25) consisting of the 25-kDa Plasmodium falciparum surface protein (Pfs25) fused to the β-subunit of a cholera toxin (CtxB) is produced inside the cells can be used as oral vaccines for malaria.

However, in the related art above, even when a target substance is produced inside the cells of the genetically modified strain of a photosynthetic microorganism, the substance is not secreted out of the cells and accumulates therein or unlikely to be secreted out of the cells in an efficient manner. Therefore, it is necessary to crush the cells in order to collect the target substance. Thus, matter production requires a lot of effort and time. Moreover, since a variety of substances are present inside the cells, it may become necessary to remove those substances for purifying the target substance. This results in a reduction in the collection rate of the target substance. Furthermore, it is necessary to prepare a new genetically modified strain each time when a target substance is produced. This increases the effort and time required and the production costs. As described above, in the above-described related art, the efficiencies of matter production using photosynthetic microorganisms are still at low levels. Thus, the development of technologies with which further high production efficiencies can be achieved has been anticipated.

The structure of the cell wall and cell membrane of cyanobacteria has low permeability to substances produced inside the cells. It is not easy to artificially modify the structure of the cell wall and cell membrane in order to enhance the ability of secretory matter production. In particular, substances having high molecular weights (referred to also as "high-molecular-weight compounds"), such as proteins, are unlikely to be secreted out of the cells, unlike substances having relatively low molecular weights (referred to also as "low-molecular-weight compounds"), such as amino acids.

It is described that deletion of the slr1841 or slr0688 gene, which is responsible for the adhesion between the outer membrane and cell wall of cyanobacteria and which contributes to the structural stability of the cell surface layer, results in the loss of the proliferating ability of cyanobacterial cells (NPL 6: Hikaru Kowata, Studies on molecular basis of cyanobacterial outer membrane function and its evolutionary relationship with primitive chloroplasts, Doctoral dissertation, [Online], 2018.03.27, Internet: <URL: http://hdl.handle.net/10097/00122689> and NPL 7: Seiji Kojima, Clarification and application of membrane stabilizing mechanism and substance permeation mechanism derived from bacteria that act on the chloroplast surface membrane, KAKENHI (Grants-in-Aid for Scientific Research), [Online], 2018.04.23, Internet: <URL: https://kaken.nii.ac.jp/grant/KAKENHI-PROJECT-18H02117>).

As a technology that addresses the above-described issues, PTL 1 discloses a modified cyanobacterium in which the function of a protein responsible for the bonding between the outer membrane and cell wall of a cyanobacterium (hereinafter, such a protein is referred to also as "bond-related protein") is suppressed or lost and a method for producing a protein using the modified cyanobacterium. In the technology described in PTL 1, the outer membrane of the modified cyanobacterium is deprived from the cell wall while the cell proliferating ability is maintained. Therefore, when the cells are cultivated, proteins produced inside the cells are secreted out of the cells. This enables efficient production of proteins.

However, in the above related art, it is necessary to determine whether the conditions of the cells of the modified cyanobacterium are suitable for efficient matter production by inspecting the cells with an electron microscope or using a complex biochemical analytical method, which requires a lot of effort and time.

It is known that the matter productivity is enhanced when the outer membrane of a cyanobacterium is deprived from the cell wall. However, it is necessary to perform inspection with an electron microscope or use a complex biochemical analytical method for determining the deprivation of the outer membrane; at the present time, there is no easy and simple method available.

Accordingly, the present inventor conducted extensive studies of a method for determining whether the outer membrane is deprived from the cell wall in an easy and simple manner, in order to determine whether the conditions of cyanobacterial cells are suitable for efficient matter production. As a result, the inventor found that it is possible to determine whether the outer membrane of a cyanobacterium is deprived from the cell wall in an easy and simple manner on the basis of the concentrations of specific amino acids in a culture supernatant of the cyanobacterium. Thus, according to the present disclosure, it becomes possible to determine whether a cyanobacterium is suitable for efficient matter production. This enhances the matter productivity of a cyanobacterium.

(Summary of the Present Disclosure)

The summary of the aspects of the present disclosure is as follows.

A method for determining deprivation of an outer membrane of a cyanobacterium according to an aspect of the present disclosure includes a measurement step in which a concentration of at least one amino acid selected from the group consisting of isoleucine, leucine, tyrosine, and phenylalanine in a culture supernatant of a cyanobacterium is measured, and a determination step in which whether an outer membrane of the cyanobacterium is deprived from a cell wall of the cyanobacterium is determined on the basis of the concentration of the at least one amino acid, the concentration being measured in the measurement step.

In the method for determining deprivation of an outer membrane of a cyanobacterium, it is only necessary to collect a culture supernatant of a cyanobacterium and measure the concentration of at least one amino acid selected from the group consisting of the four amino acids above in the culture supernatant. Thus, the deprivation of the outer membrane of the cyanobacterium can be determined in an easy and simple manner.

For example, in the method for determining deprivation of an outer membrane of a cyanobacterium according to an aspect of the present disclosure, in the measurement step, concentrations of four amino acids, that is, isoleucine, leucine, tyrosine, and phenylalanine, may be measured and, in the determination step, whether the outer membrane of the cyanobacterium is deprived from the cell wall of the cyanobacterium may be determined on the basis of the concentrations of the four amino acids.

In the above method for determining deprivation of an outer membrane of a cyanobacterium, the concentrations of the four amino acids in the culture supernatant of the cyanobacterium are measured and the deprivation of the outer membrane of the cyanobacterium is determined on the basis of the concentrations of the amino acids. This makes it possible to determine the deprivation of the outer membrane of the cyanobacterium with further high accuracy.

For example, in the method for determining deprivation of an outer membrane of a cyanobacterium according to an aspect of the present disclosure, in the determination step, it may be determined that the outer membrane of the cyanobacterium is deprived from the cell wall when at least one of the concentration of the at least one amino acid is equal to or more than a threshold, and it may be determined that the outer membrane of the cyanobacterium is not deprived from the cell wall when all of the concentration of the at least one amino acid is less than the threshold.

In the above method for determining deprivation of an outer membrane of a cyanobacterium, it is determined that the outer membrane of the cyanobacterium is deprived when even one of the concentration of the at least one amino acid is more than the threshold. Thus, even when fluctuations occur due to the cultivation conditions of the cyanobacterium, the deprivation of the outer membrane of the cyanobacterium can be determined with high accuracy.

For example, in the method for determining deprivation of an outer membrane of a cyanobacterium according to an aspect of the present disclosure, the threshold may be 100 nM.

With the above method for determining deprivation of an outer membrane of a cyanobacterium, the deprivation of the outer membrane of the cyanobacterium can be determined quantitatively in an easy and simple manner on the basis of whether the concentration of at least one amino acid selected from the group consisting of the four amino acids in the culture supernatant of the cyanobacterium the outer membrane of which has been deprived from the cell wall is more than 100 nM.

An apparatus for determining deprivation of an outer membrane of a cyanobacterium according to an aspect of the present disclosure includes a measurement unit that measures a concentration of at least one amino acid selected from the group consisting of isoleucine, leucine, tyrosine, and phenylalanine in a culture supernatant of a cyanobacterium, and a determination unit that determines whether an outer membrane of the cyanobacterium is deprived from a cell wall of the cyanobacterium on the basis of the concentration of the at least one amino acid, the concentration being measured by the measurement unit.

In the apparatus for determining deprivation of an outer membrane of a cyanobacterium, it is only necessary to measure the concentration of at least one amino acid selected from the group consisting of the four amino acids above in a culture supernatant of the cyanobacterium. Thus, the deprivation of the outer membrane of cyanobacterium can be determined in an easy and simple manner.

A program according to an aspect of the present disclosure is a program for causing a computer to execute a method for determining whether an outer membrane of a cyanobacterium is deprived from a cell wall of the cyanobacterium on the basis of a concentration of at least one amino acid selected from the group consisting of isoleucine, leucine, tyrosine, and phenylalanine in a culture supernatant of the cyanobacterium.

The above program causes a computer to determine whether the outer membrane of a cyanobacterium is deprived from the cell wall on the basis of the concentration of at least one amino acid selected from the group consisting of the four amino acids in a culture supernatant of the cyanobacterium. Thus, the computer can determine the deprivation of the outer membrane of the cyanobacterium in an easy and simple manner.

Embodiments are described specifically with reference to the attached drawings below.

It should be noted that the embodiments described below are all comprehensive or specific examples. The values, materials, steps, the order of the steps, and the like described in the following embodiments are intended to be illustrative but not restrictive of the present disclosure. Among the elements described in the following embodiments, the elements not described in the independent claims, which indicate the highest concept, are described as optional elements.

The attached drawings are not always exact. In the drawings, substantially the same structures are denoted by the same reference numeral, and the duplicate description thereof may be omitted or simplified.

Hereinafter, a numerical range means not only a strict meaning but also, for example, a substantially equivalent range, such as the amount (e.g., number or concentration) of a protein, or measuring the above range.

The terms "bacterial cell" and "cell" used herein both refer to an individual cyanobacterium.

(Embodiments)

[1. Definitions]

In the description, the identity between base sequences or amino acid sequences is calculated using the BLAST (basic local alignment search tool) algorithm. Specifically, the above identity is calculated by a pairwise analysis using the BLAST program available in the website (https://blast.ncbi.nlm.nih.gov/Blast.cgi) of NCBI (National Center for Biotechnology Information). Information regarding the genes of cyanobacteria and information regarding the proteins coded by the genes are published on, for example, the NCBI database above and Cyanobase (http://genome.microbedb.jp/cyanobase/). The amino acid sequences of target proteins and the base sequences of the genes coding the proteins can be acquired from the database.

[2. Apparatus for Determining Deprivation of Outer Membrane of Cyanobacterium]

An apparatus for determining deprivation of the outer membrane of a cyanobacterium according to the embodiment (hereinafter, referred to simply as "determination apparatus") is described below. Fig. 1 is a block diagram illustrating an example of the functional structure of an apparatus for determining deprivation of the outer membrane of a cyanobacterium according to the embodiment.

As illustrated in Fig. 1, a determination apparatus 100 includes, for example, a measurement unit 110, a controller 120, a memory 130, an input receiving unit 140, and a display 150. The controller 120 includes, for example, a determination unit 122.

The measurement unit 110 measures, for example, the concentration of at least one amino acid selected from the group consisting of isoleucine, leucine, tyrosine, and phenylalanine in a culture supernatant of a cyanobacterium. The measurement unit 110 may be any measurement unit with which the concentration of an amino acid in the culture supernatant can be measured and may be an amino acid analyzer known in the related art. The measurement unit 110 is, for example, liquid chromatography (HPLC), a liquid chromatography-mass spectrometer (LC/MS), or a liquid chromatography-tandem mass spectrometer (LC-MS/MS).

Although not illustrated in the drawing, the measurement unit 110 includes a controller that controls, for example, the action of the measurement unit 110. The controller controls the action of the measurement unit 110 in accordance with a control signal sent from the controller 120 of the determination apparatus 100. Although the determination apparatus 100 includes a measurement unit 110 in the example illustrated in Fig. 1, the determination apparatus 100 does not necessarily include the measurement unit 110. In such a case, the measurement unit 110 is a measurement device, and the determination apparatus 100 is connected to the measurement device by communications.

The controller 120 performs information processing for controlling the action of the determination apparatus 100. The controller 120 is implemented by a microcomputer and may be implemented by a processor or a dedicated circuit. Specifically, the controller 120 includes a determination unit 122. The determination unit 122 is implemented by the processor executing a program for the information processing.

The determination unit 122 determines whether the outer membrane of the cyanobacterium is deprived from the cell wall on the basis of the concentration of at least one amino acid which has been measured by the measurement unit 110. For example, the function of the determination unit 122 is implemented by a CPU (central processing unit) executing a program stored in the memory 130. The specific function of the determination unit 122 is described in the next section.

The memory 130 is a storage device that stores, for example, a control program executed by the controller 120. The memory 130 is implemented by, for example, a semiconductor memory.

The input receiving unit 140 receives a command input by the user. Specifically, the input receiving unit 140 is implemented by a mouse, a microphone, a touch panel, or the like.

The display 150 is a display device that displays information for the user on the basis of the control by the controller 120. The display 150 is implemented by a liquid crystal panel or an organic EL (electroluminescence) panel.

[3. Method for Determining Deprivation of Outer Membrane of Cyanobacterium]

A method for determining deprivation of the outer membrane of a cyanobacterium according to the embodiment is described with reference to Figs. 2A and 2B below. Fig. 2A is a flowchart illustrating an example of the flow of a method for determining deprivation of the outer membrane of a cyanobacterium according to the embodiment. Fig. 2B is a flowchart illustrating the detailed flow of Step S02 illustrated in Fig. 2A.

The above determination method is implemented using the above-described determination apparatus. For example, as illustrated in Fig. 2A, the measurement unit of the determination apparatus measures the concentration of at least one amino acid selected from the group consisting of isoleucine, leucine, tyrosine, and phenylalanine in a culture supernatant of a cyanobacterium introduced into the measurement unit (S01). The culture supernatant may be sampled at predetermined intervals (e.g., on a daily basis) after the main cultivation has been started. Sampling may be done manually by the user or automatically. Isoleucine, leucine, tyrosine, and phenylalanine are typically secreted into the culture supernatant in the case where cyanobacterial cells the outer membrane of which has been deprived are cultivated.

The determination unit of the determination apparatus determines whether the outer membrane of the cyanobacterium is deprived from the cell wall on the basis of the concentration of the amino acid which is measured by the measuring unit (S02). Specifically, as illustrated in Fig. 2B, in Step S02, the determination unit determines whether all of the concentrations of the amino acids which are measured by the measurement unit are less than the threshold (S11). When it is determined that all of the concentrations of the amino acids are less than the threshold ("Yes" in S11), the determination unit determines that the outer membrane of the cyanobacterium is not deprived from the cell wall (S12).

On the other hand, when at least one of the concentrations of the amino acids which are measured by the measurement unit is equal to or more than the threshold ("No" in S11), the determination unit determines that the outer membrane of the cyanobacterium is deprived from the cell wall (S13).

In the method for determining deprivation of the outer membrane of a cyanobacterium, whether the outer membrane of a cyanobacterium is deprived from the cell wall is determined by measuring (i.e., quantifying) the concentrations of isoleucine, leucine, tyrosine, and phenylalanine in a culture supernatant of the cyanobacterium. However, the amino acids and biomolecules (e.g., intracellular metabolites) that are to be analyzed are not limited to these.

The measuring method conducted in Step S01 may be any method by which amino acids or biomolecules can be analyzed and may be a method performed using liquid chromatography (HPLC), a liquid chromatography-mass spectrometer (LC/MS), a liquid chromatography-tandem mass spectrometer (LC-MS/MS), or the like. The measuring method conducted in Step S01 may be, for example, an enzyme assay for detecting specific amino acids or biomolecules.

[4. Cyanobacterium]

Cyanobacteria are referred to also as "blue-green algae" or "blue-green bacteria". Cyanobacteria are a group of prokaryotes which contain chlorophyll that captures light energy and electrolyze water using the energy to generate oxygen, that is, perform photosynthesis. There are a variety of types of cyanobacteria. Examples of the cyanobacteria include, focusing on cell shape, unicellular cyanobacteria, such as Synechocystis sp. PCC 6803; and multicellular, filamentous cyanobacteria, such as Anabaena sp. PCC 7120. Examples of the cyanobacteria include, focusing on growing environment, thermophilic cyanobacteria, such as Thermosynechococcus elongatus; oceanic cyanobacteria, such as Synechococcus elongatus; and freshwater cyanobacteria, such as Synechocystis. There are various types of cyanobacteria having unique characteristics. Examples thereof include cyanobacteria that have gas vacuoles and produce toxins, such as Microcystis aeruginosa; and Gloeobacter violaceus, which does not have thylakoids and has a protoplasmic membrane including a protein referred to as "phycobilisome" which serves as a light-harvesting antenna.

Fig. 3 is a diagram schematically illustrating the surface layer of a cyanobacterial cell. As illustrated in Fig. 3, the surface layer of a cyanobacterial cell is constituted by, in order from inside, a plasma membrane (referred to also as "inner membrane 1"), a peptidoglycan 2, and an outer membrane 5, which is a lipid membrane that forms an outermost layer of the cell. The peptidoglycan 2 is provided with sugar chains 3 composed of glucosamine, mannosamine, or the like, which are covalently bonded to the peptidoglycan 2. Pyruvic acid is bonded to the covalently bonded sugar chains 3 (NPL 8: Jurgens and Weckesser, 1986, J. Bacteriol., 168:568-573). In the description, the peptidoglycan 2 and the covalently bonded sugar chains 3 are referred to collectively as "cell wall 4". The gap between the plasma membrane (i.e., inner membrane 1) and the outer membrane 5 is referred to as "periplasm", in which various enzymes responsible for protein degradation or formation of three-dimensional structures, degradation of lipid or nucleic acid, incorporation of extracellular nutrients, or the like are present.

An SLH domain-containing outer membrane protein (e.g., Slr1841 in the drawing) includes a C-end-side region buried in a lipid membrane (also referred to as "outer membrane 5") and an N-end-side SLH domain 7 protruded from the lipid membrane. This protein is broadly distributed in cyanobacteria and germs belonging to Negativicutes, which is a group of Gram-negative bacteria (NPL 9: Kojima et al., 2016, Biosci. Biotech. Biochem., 10:1954-1959). The region buried in the lipid membrane (i.e., the outer membrane 5) forms a channel through which hydrophilic substances can permeate the outer membrane, while the SLH domain 7 is capable of binding to the cell wall 4 (NPL 10: Kowata et al., 2017, J. Bacteriol., 199:e00371-17). For the SLH domain 7 binding to the cell wall 4, the covalently bonded sugar chains 3 present in the peptidoglycan 2 need to be modified with pyruvic acid (NPL 11: Kojima et al., 2016, J. Biol. Chem., 291:20198-20209). Examples of genes coding the SLH domain-containing outer membrane protein 6 include slr1841 and slr1908 held by Synechocystis sp. PCC 6803 and oprB held by Anabaena sp. 90.

An enzyme that catalyzes the modification reaction of the covalently bonded sugar chains 3 included in the peptidoglycan 2 with pyruvic acid (hereinafter, this enzyme is referred to as "cell wall-pyruvic acid modification enzyme 9") is identified using Bacillus anthracis, which is a Gram-positive bacterium, and is named "CsaB" (NPL 12: Mesnage et al., 2000, EMBO J., 19:4473-4484). A number of types of cyanobacteria the genomic base sequences of which are known hold a gene coding a homologous protein the amino acid sequence of which is 30% or more identical to that of CsaB. Examples thereof include slr0688 held by Synechocystis sp. PCC 6803 and synpcc7502_03092 held by Synechococcus sp. 7502.

In a cyanobacterium, $CO_2$ fixed by photosynthesis is converted into various types of amino acids through a multi-step enzymatic reaction, and proteins are synthesized inside the cytoplasm of the cyanobacterium using the amino acids as raw materials. Some of the proteins work inside the cytoplasm, while others are transported from the cytoplasm to the periplasm and work inside the periplasm. However, a type of cyanobacteria which aggressively secretes proteins out of the cells has not been reported up to the present time.

Since cyanobacteria have a high photosynthetic ability, it is not always necessary to incorporate external organic substances as nutrients. Therefore, the amount of channel proteins permeable to organic substances which are included in the outer membrane 5, such as the organic substance channel protein 8 (e.g., Slr1270) illustrated in Fig. 3, is considerably small. For example, in Synechocystis sp. PCC 6803, the amount of the organic substance channel protein 8 permeable to organic substances is only about 4% of the total amount of proteins included in the outer membrane 5. On the other hand, the outer membrane 5 of cyanobacteria includes a large amount of ion channel proteins permeable to only inorganic ions, such as the SLH domain-containing outer membrane protein 6 (e.g., Slr1841) illustrated in Fig. 3, in order to incorporate inorganic ions necessary for growth into the cells with high efficiency. For example, in Synechocystis sp. PCC 6803, the amount of the ion channel protein permeable to inorganic ion is about 80% of the total amount of proteins included in the outer membrane 5.

Thus, it is considered that, since the amount of channels permeable to organic substances, such as proteins, which are present in the outer membrane 5 of a cyanobacterium, is considerably small, it is difficult to aggressively secrete the proteins produced inside the bacterial cells out of the bacterial cells. While the structure of the cell wall and cell membrane of cyanobacteria governs protein permeability, it is not easy to artificially modify the structure of the cell membrane and cell wall to enhance the ability of secretory protein production. For example, in NPL 6 and NPL 7, it is described that deletion of the slr1841 gene or slr0688 gene, which is responsible for adhesion between the outer membrane and the cell wall and contributes to the structural stability of cell surface layer results in the loss of the proliferating ability of the cells.

[5. Modified Cyanobacterium]

A cyanobacterium according to the embodiment (hereinafter, referred to as "modified cyanobacterium") is described with reference to Fig. 3 below. Note that, hereinafter, a protein is described as an example of the substance produced using the modified cyanobacterium.

In the modified cyanobacterium according to the embodiment, the function of a protein (i.e., "bond-related protein") responsible for the bonding between the outer membrane 5 and the cell wall 4 in the cyanobacterium is suppressed or lost. Specifically, for example, in the modified cyanobacterium, the total amount of proteins (i.e., bond-related proteins) responsible for the bonding between the outer membrane 5 and the cell wall 4 in the cyanobacterium is reduced to 30% or more and 70% or less of the total amount of the proteins included in the parent strain (i.e., parent cyanobacterium). Note that, for example, the expression "the total amount of bond-related proteins is reduced to 30% of the total amount of proteins included in the parent strain" means that 70% of the total amount of proteins included in the parent strain are lost while 30% of the total amount of proteins remain. As a result, in the modified cyanobacterium, the degree of bonding (e.g., amount of bonds and bonding power) between the outer membrane 5 and the cell wall 4 is partially reduced, and the outer membrane 5 is likely to partially detach from the cell wall 4 accordingly. This increases the secretory protein productivity of the modified cyanobacterium with which the proteins produced inside the bacterial cells are secreted out of the bacterial cells. In addition, since it is not necessary to crush the bacterial cells to collect the proteins, the modified cyanobacterium can be repeatedly used to produce the proteins even after the proteins have been collected. Note that, in the description, the action of the modified cyanobacterium to create proteins inside the bacterial cells is referred to as "production", and an action to secrete the proteins out of the bacterial cells is referred to as "secretory production".

The protein responsible for the bonding between the outer membrane 5 and the cell wall 4 may be, for example, at least one of the SLH domain-containing outer membrane protein 6 or the cell wall-pyruvic acid modification enzyme 9. In the modified cyanobacterium according to the embodiment, for example, the function of at least one protein selected from the SLH domain-containing outer membrane protein 6 and the cell wall-pyruvic acid modification enzyme 9 is suppressed. For example, in the modified cyanobacterium, (i) at least one function of the SLH domain-containing outer membrane protein 6 and the cell wall-pyruvic acid modification enzyme 9 may be suppressed, and (ii) at least one of the expression of the SLH domain-containing outer membrane protein 6 that binds to the cell wall 4 or the expression of the enzyme (i.e., cell wall-pyruvic acid modification enzyme 9) that catalyzes the modification reaction of the sugar chains bonded to the surface of the cell wall 4 with pyruvic acid may be suppressed. This reduces the degree of bonding (i.e., amount of bonds and bonding power) between the SLH domain 7 of the SLH domain-containing outer membrane protein 6 included in the outer membrane 5 and the covalently bonded sugar chains 3 present on the surface of the cell wall 4. Accordingly, the likelihood of the outer membrane 5 detaching from the cell wall 4 is increased at the portion in which the degree of the above bonding is reduced. As a result of the outer membrane 5 partially detaching from the cell wall 4, the likelihood of the proteins present inside the cells of the modified cyanobacterium and, in particular, inside the periplasm of the modified cyanobacterium leaking out of the cells (out of the outer membrane 5) is increased.

As described above, since cyanobacteria have a high photosynthetic ability, it is not always necessary to incorporate external organic substances as nutrients. Only light, air, water, and a trace amount of inorganic substances are necessary for cultivating cyanobacteria. Cyanobacteria incorporate inorganic substances into the cells through the ion channels present in the outer membrane 5 and produce proteins inside the cells. In particular, various types of proteins are present in the periplasm, which is the gap between the outer membrane 5 and the cell wall 4. In the modified cyanobacterium according to the embodiment, the function of the protein responsible for the bonding between the outer membrane 5 and the cell wall 4 is suppressed. This increases the likelihood of the outer membrane 5 being partially deprived from the cell wall 4. The proteins present inside the periplasm are leaked from the deprived portion of the outer membrane 5 into the culture solution. Thus, in the modified cyanobacterium, the secretory protein productivity, with which the proteins produced inside the bacterial cells are secreted out of the bacterial cells, is enhanced.

A cyanobacterium modified such that the outer membrane 5 partially detaches from the cell wall 4 as a result of the function of at least one bond-related protein selected from the SLH domain-containing outer membrane protein 6 and the cell wall-pyruvic acid modification enzyme 9 being suppressed is described specifically below.

The type of the cyanobacterium in which at least one of the expression of the SLH domain-containing outer membrane protein 6 or the expression of the cell wall-pyruvic acid modification enzyme 9 has not been suppressed, which serves as the parent microorganism of the modified cyanobacterium according to the embodiment (hereinafter, this cyanobacterium is referred to as "parent strain" or "parent cyanobacterium"), is not limited. The parent cyanobacterium may be any type of cyanobacterium. For example, the parent cyanobacterium may be Synechocystis, Synechococcus, Anabaena, or Thermosynechococcus and may be, in particular, Synechocystis sp. PCC 6803, Synechococcus sp. PCC 7942, or Thermosynechococcus elongatus BP-1. The parent strain may be any cyanobacterium in which the total amount of the bond-related proteins has not been reduced to 30% or more and 70% or less; the parent strain may be a wild-type strain and may be a modified strain including the bond-related proteins in substantially the same amount as a wild-type strain.

The amino acid sequences of the SLH domain-containing outer membrane protein 6 and the enzyme (i.e., cell wall-pyruvic acid modification enzyme 9) that catalyzes the cell wall-pyruvic acid modification reaction which are present in the parent cyanobacterium, the base sequences of genes coding the above bond-related proteins, and the positions of the genes in the chromosome DNA or plasmid can be confirmed on the above-described NCBI database and Cyanobase.

Note that the SLH domain-containing outer membrane protein 6 and the cell wall-pyruvic acid modification enzyme 9, the functions of which are suppressed in the modified cyanobacterium according to the embodiment, may be the SLH domain-containing outer membrane protein and the cell wall-pyruvic acid modification enzyme of any parent cyanobacterium and are not limited depending on the location (e.g., chromosome DNA or plasmid) at which genes coding the SLH domain-containing outer membrane protein and the cell wall-pyruvic acid modification enzyme are present.

For example, in the case where the parent cyanobacterium is Synechocystis, the SLH domain-containing outer membrane protein 6 may be, for example, Slr1841, Slr1908, or Slr0042. In the case where the parent cyanobacterium is Synechococcus, the SLH domain-containing outer membrane protein 6 may be, for example, NIES970_09470. In the case where the parent cyanobacterium is Anabaena, the SLH domain-containing outer membrane protein 6 may be, for example, Anacy_5815 or Anacy_3458. In the case where the parent cyanobacterium is Microcystis, the SLH domain-containing outer membrane protein 6 may be, for example, A0A0F6U6F8_MICAE. In the case where the parent cyanobacterium is Cyanothece, the SLH domain-containing outer membrane protein 6 may be, for example, A0A3B8XX12_9CYAN. In the case where the parent cyanobacterium is Leptolyngbya, the SLH domain-containing outer membrane protein 6 may be, for example, A0A1Q8ZE23_9CYAN. In the case where the parent cyanobacterium is Calothrix, the SLH domain-containing outer membrane protein 6 may be, for example, A0A1Z4R6U0_9CYAN. In the case where the parent cyanobacterium is Nostoc, the SLH domain-containing outer membrane protein 6 may be, for example, A0A1C0VG86_9NOSO. In the case where the parent cyanobacterium is Crocosphaera, the SLH domain-containing outer membrane protein 6 may be, for example, B1WRN6_CROS5. In the case where the parent cyanobacterium is Pleurocapsa, the SLH domain-containing outer membrane protein 6 may be, for example, K9TAE4_9CYAN.

Specifically, the SLH domain-containing outer membrane protein 6 may be, for example, Slr1841 (SEQ ID NO: 1) of Synechocystis sp. PCC 6803, NIES970_09470 (SEQ ID NO: 2) of Synechococcus sp. NIE S-970, or Anacy_3458 (SEQ ID NO: 3) of Anabaena cylindrica PCC 7122. The SLH domain-containing outer membrane protein 6 may also be a protein the amino acid sequence of which is 50% or more identical to that of the SLH domain-containing outer membrane protein 6.

Thus, in the modified cyanobacterium, for example, (i) the function of any of the SLH domain-containing outer membrane proteins 6 represented by SEQ ID NOs: 1 to 3 above or a protein the amino acid sequence of which is 50% or more identical to that of any of the above SLH domain-containing outer membrane proteins 6 may be suppressed and (ii) the expression of any of the SLH domain-containing outer membrane proteins 6 represented by SEQ ID NOs: 1 to 3 above or the expression of a protein the amino acid sequence of which is 50% or more identical to that of any of the above SLH domain-containing outer membrane proteins 6 may be suppressed. Therefore, in the modified cyanobacterium, (i) the function of the SLH domain-containing outer membrane protein 6 present in the outer membrane 5 or a protein having substantially the same function as the SLH domain-containing outer membrane protein 6 is suppressed, or (ii) the amount of expression of the SLH domain-containing outer membrane protein 6 present in the outer membrane 5 or a protein having substantially the same function as the SLH domain-containing outer membrane protein 6 is reduced. Therefore, in the modified cyanobacterium according to the embodiment, the amount of bonds and bonding power with which the bond domain (e.g., SLH domain 7) for bonding between the outer membrane 5 and the cell wall 4 binds to the cell wall 4 are reduced and, consequently, the likelihood of the outer membrane 5 partially detaching from the cell wall 4 is increased.

In general, it is considered that proteins are highly likely to have substantially the same function when the identity between the amino acid sequences of the proteins is 30% or more because, in such a case, the homology between the three-dimensional structures of the proteins is high. Therefore, the SLH domain-containing outer membrane protein 6 the function of which is suppressed may be, for example, a protein or polypeptide that is composed of an amino acid sequence that is 40% or more, preferably 50% or more, more preferably 60% or more, further preferably 70% or more, still more preferably 80% or more, and still further preferably 90% or more identical to the amino acid sequence of any of the SLH domain-containing outer membrane proteins 6 represented by SEQ ID NOs: 1 to 3 above and that is capable of binding to the covalently bonded sugar chains 3 present on the cell wall 4.

For example, in the case where the parent cyanobacterium is Synechocystis, the cell wall-pyruvic acid modification enzyme 9 may be, for example, Slr0688. In the case where the parent cyanobacterium is Synechococcus, the cell wall-pyruvic acid modification enzyme 9 may be, for example, Syn7502_03092 or Synpcc7942_1529. In the case where the parent cyanobacterium is Anabaena, the cell wall-pyruvic acid modification enzyme 9 may be, for example, ANA_C20348 or Anacy_1623. In the case where the parent cyanobacterium is Microcystis, the cell wall-pyruvic acid modification enzyme 9 may be, for example, CsaB (NCBI Access ID: TRU80220). In the case where the parent cyanobacterium is Cyanothece, the cell wall-pyruvic acid modification enzyme 9 may be, for example, CsaB (NCBI Access ID: WP_107667006.1). In the case where the parent cyanobacterium is Spirulina, the cell wall-pyruvic acid modification enzyme 9 may be, for example, CsaB (NCBI Access ID: WP_026079530.1). In the case where the parent cyanobacterium is Calothrix, the cell wall-pyruvic acid modification enzyme 9 may be, for example, CsaB (NCBI Access ID: WP_096658142.1). In the case where the parent cyanobacterium is Nostoc, the cell wall-pyruvic acid modification enzyme 9 may be, for example, CsaB (NCBI Access ID: WP_099068528.1). In the case where the parent cyanobacterium is Crocosphaera, the cell wall-pyruvic acid modification enzyme 9 may be, for example, CsaB (NCBI Access ID: WP_012361697.1). In the case where the parent cyanobacterium is Pleurocapsa, the cell wall-pyruvic acid modification enzyme 9 may be, for example, CsaB (NCBI Access ID: WP_036798735).

Specifically, the cell wall-pyruvic acid modification enzyme 9 may be, for example, Slr0688 (SEQ ID NO: 4) of Synechocystis sp. PCC 6803, Synpcc7942_1529 (SEQ ID NO: 5) of Synechococcus sp. PCC 7942, or Anacy_1623 (SEQ ID NO: 6) of Anabaena cylindrica PCC 7122. The cell wall-pyruvic acid modification enzyme 9 may also be a protein the amino acid sequence of which is 50% or more identical to that of the cell wall-pyruvic acid modification enzyme 9.

Thus, in the modified cyanobacterium, for example, (i) the function of any of the cell wall-pyruvic acid modification enzymes 9 represented by SEQ ID NOs: 4 to 6 above or a protein the amino acid sequence of which is 50% or more identical to that of any of the above cell wall-pyruvic acid modification enzyme 9 is suppressed or (ii) the expression of any of the cell wall-pyruvic acid modification enzymes 9 represented by SEQ ID NOs: 4 to 6 above or the expression of a protein the amino acid sequence of which is 50% or more identical to that of any of the above cell wall-pyruvic acid modification enzymes 9 is suppressed. Therefore, in the modified cyanobacterium, (i) the function of the cell wall-pyruvic acid modification enzyme 9 or a protein having substantially the same function as the enzyme is suppressed, or (ii) the amount of expression of the cell wall-pyruvic acid modification enzyme 9 or a protein having substantially the same function as the enzyme is reduced. This reduces the likelihood of the covalently bonded sugar chains 3 present on the surface of the cell wall 4 being modified with pyruvic acid. As a result, the amount and power of bonding between the sugar chains 3 present on the cell wall 4 and the SLH domain 7 of the SLH domain-containing outer membrane protein 6 present in the outer membrane 5 are reduced. Thus, in the modified cyanobacterium according to the embodiment, the likelihood of the covalently bonded sugar chains 3 present on the surface of the cell wall 4 being modified with pyruvic acid is reduced, the power of bonding between the cell wall 4 and the outer membrane 5 is reduced accordingly, and, consequently, the likelihood of the outer membrane 5 partially detaching from the cell wall 4 is increased.

As described above, it is considered that proteins are highly likely to have substantially the same function when the identity between the amino acid sequences of the proteins is 30% or more. Therefore, the cell wall-pyruvic acid modification enzyme 9 the function of which is suppressed may be, for example, a protein or polypeptide that is composed of an amino acid sequence that is 40% or more, preferably 50% or more, more preferably 60% or more, further preferably 70% or more, still more preferably 80% or more, and still further preferably 90% or more identical to the amino acid sequence of any of the cell wall-pyruvic acid modification enzymes 9 represented by SEQ ID NOs: 4 to 6 above and that is capable of catalyzing the modification reaction of the covalently bonded sugar chains 3 of the peptidoglycan 2 of the cell wall 4 with pyruvic acid.

Note that the expression "suppress the function of the SLH domain-containing outer membrane protein 6" used herein refers to suppressing the ability of the protein to bind to the cell wall 4, limiting or disabling the transportation of the protein to the outer membrane 5, or suppressing the ability of the protein which works when the protein is buried in the outer membrane 5.

Note that the expression "suppress the function of the cell wall-pyruvic acid modification enzyme 9" used herein refers to suppressing the ability of the protein to modify the covalently bonded sugar chains 3 present in the cell wall 4 with pyruvic acid.

The method for suppressing the functions of the above proteins is not limited and may be any method commonly used for suppressing protein functions. The above method may be, for example, deleting or inactivating the gene coding the SLH domain-containing outer membrane protein 6 and the gene coding the cell wall-pyruvic acid modification enzyme 9, inhibiting the transcription of the above genes, inhibiting the translation of the transcriptional products of the genes, or administering an inhibitor that specifically inhibits the above proteins.

In the modified cyanobacterium according to the embodiment, a gene responsible for the expression of the protein responsible for the bonding between the outer membrane 5 and the cell wall 4 may be deleted or inactivated. In such a case, in the modified cyanobacterium, the expression of a protein responsible for the bonding between the cell wall 4 and the outer membrane 5 is suppressed or the function of the protein is suppressed. This partially reduces the degree of bonding (i.e., amount of bonds and bonding power) between the cell wall 4 and the outer membrane 5. As a result, in the modified cyanobacterium, the outer membrane 5 is likely to partially detach from the cell wall 4, and the likelihood of the proteins produced inside the bacterial cells leaking out of the outer membrane 5, that is, out of the bacterial cell, is increased. This increases the secretory protein productivity of the modified cyanobacterium according to the embodiment with which the proteins produced inside the bacterial cells are secreted out of the bacterial cells. In addition, since it is not necessary to crush the bacterial cells to collect the proteins, the modified cyanobacterium can be repeatedly used to produce the proteins even after the proteins have been collected.

The gene responsible for the expression of the protein responsible for the bonding between the outer membrane 5 and the cell wall 4 may be, for example, at least one of the gene coding the SLH domain-containing outer membrane protein 6 or the gene coding the cell wall-pyruvic acid modification enzyme 9. In the modified cyanobacterium, at least one gene selected from the gene coding the SLH domain-containing outer membrane protein 6 and the gene coding the cell wall-pyruvic acid modification enzyme 9 is deleted or inactivated. Therefore, in the modified cyanobacterium, for example, (i) the expression of at least one of the SLH domain-containing outer membrane protein 6 or the cell wall-pyruvic acid modification enzyme 9 is suppressed or (ii) the function of at least one of the SLH domain-containing outer membrane protein 6 or the cell wall-pyruvic acid modification enzyme 9 is suppressed. This reduces the degree of bonding (i.e., amount of bonds and bonding power) between the SLH domain 7 of the SLH domain-containing outer membrane protein 6 included in the outer membrane 5 and the covalently bonded sugar chains 3 present on the surface of the cell wall 4. This increases the likelihood of the outer membrane 5 detaching from the cell wall 4 at the portion in which the degree of bonding between the outer membrane 5 and the cell wall 4 is reduced. Thus, in the modified cyanobacterium according to the embodiment, the likelihood of the outer membrane 5 partially detaching from the cell wall 4 is increased as a result of the reduction in the degree of bonding between the outer membrane 5 and the cell wall 4. This increases the likelihood of the proteins produced inside the bacterial cells leaking out of the bacterial cells.

In the present embodiment, for example, the transcription of at least one of the gene coding the SLH domain-containing outer membrane protein 6 or the gene coding the cell wall-pyruvic acid modification enzyme 9 may be suppressed in order to suppress the function of at least one of the SLH domain-containing outer membrane protein 6 or the cell wall-pyruvic acid modification enzyme 9 in the cyanobacterium.

For example, in the case where the parent cyanobacterium is Synechocystis, the gene coding the SLH domain-containing outer membrane protein 6 may be, for example, slr1841, slr1908, or slr0042. In the case where the parent cyanobacterium is Synechococcus, the gene coding the SLH domain-containing outer membrane protein 6 may be, for example, nies970_09470. In the case where the parent cyanobacterium is Anabaena, the gene coding the SLH domain-containing outer membrane protein 6 may be, for example, anacy_5815 or anacy _3458. In the case where the parent cyanobacterium is Microcystis, the gene coding the SLH domain-containing outer membrane protein 6 may be, for example, A0A0F6U6F8_MICAE. In the case where the parent cyanobacterium is Cyanothece, the gene coding the SLH domain-containing outer membrane protein 6 may be, for example, A0A3B8XX12_9CYAN. In the case where the parent cyanobacterium is Leptolyngbya, the gene coding the SLH domain-containing outer membrane protein 6 may be, for example, A0A1Q8ZE23_9CYAN. In the case where the parent cyanobacterium is Calothrix, the gene coding the SLH domain-containing outer membrane protein 6 may be, for example, A0A1Z4R6U0_9CYAN. In the case where the parent cyanobacterium is Nostoc, the gene coding the SLH domain-containing outer membrane protein 6 may be, for example, A0A1C0VG86_9NOSO. In the case where the parent cyanobacterium is Crocosphaera, the gene coding the SLH domain-containing outer membrane protein 6 may be, for example, B1WRN6_CROS5. In the case where the parent cyanobacterium is Pleurocapsa, the gene coding the SLH domain-containing outer membrane protein 6 may be, for example, K9TAE4_9CYAN. The base sequences of the above genes are available from the above-described NCBI database or Cyanobase.

Specifically, the gene coding the SLH domain-containing outer membrane protein 6 may be slr1841 (SEQ ID NO: 7) of Synechocystis sp. PCC 6803, nies970_09470 (SEQ ID NO: 8) of Synechococcus sp. NIES-970, anacy _3458 (SEQ ID NO: 9) of Anabaena cylindrica PCC 7122 , or a gene the base sequence of which is 50% or more identical to that of any of the above genes.

Thus, in the modified cyanobacterium, the gene coding any of the SLH domain-containing outer membrane proteins 6 represented by SEQ ID NOs: 7 to 9 or a gene the base sequence of which is 50% or more identical to that of any of the above genes is deleted or inactivated. Therefore, in the modified cyanobacterium, (i) the expression of any of the above SLH domain-containing outer membrane proteins 6 or a protein having a function equivalent to that of any of the above proteins is suppressed, or (ii) the function of any of the above SLH domain-containing outer membrane proteins 6 or a protein having a function equivalent to that of any of the above proteins is suppressed. Thus, in the modified cyanobacterium according to the embodiment, the amount of bonds and bonding power with which the bond domain (e.g., SLH domain 7) for bonding between the outer membrane 5 and the cell wall 4 binds to the cell wall 4 are reduced and, consequently, the likelihood of the outer membrane 5 partially detaching from the cell wall 4 is increased.

As described above, it is considered that proteins are highly likely to have substantially the same function when the identity between the amino acid sequences of the proteins is 30% or more. Therefore, it is considered that proteins having substantially the same function are highly likely to be expressed when the base sequences of the genes coding the proteins are 30% or more identical to each other. Therefore, the gene coding the SLH domain-containing outer membrane protein 6 the function of which is suppressed may be, for example, a gene that is composed of a base sequence that is 40% or more, preferably 50% or more, more preferably 60% or more, further preferably 70% or more, still more preferably 80% or more, and still further preferably 90% or more identical to the base sequence of any of the genes coding the SLH domain-containing outer membrane proteins 6 represented by SEQ ID NOs: 7 to 9 above and that codes a protein or polypeptide capable of binding to the covalently bonded sugar chains 3 on the cell wall 4.

For example, in the case where the parent cyanobacterium is Synechocystis, the gene coding the cell wall-pyruvic acid modification enzyme 9 may be, for example, slr0688. In the case where the parent cyanobacterium is Synechococcus, the gene coding the cell wall-pyruvic acid modification enzyme 9 may be, for example, syn7502_03092 or synpcc7942_1529. In the case where the parent cyanobacterium is Anabaena, the gene coding the cell wall-pyruvic acid modification enzyme 9 may be, for example, ana_C20348 or anacy_1623. In the case where the parent cyanobacterium is Microcystis, the gene coding the cell wall-pyruvic acid modification enzyme 9 may be, for example, csaB (NCBI Access ID: TRU80220). In the case where the parent cyanobacterium is Cynahothece, the gene coding the cell wall-pyruvic acid modification enzyme 9 may be, for example, csaB (NCBI Access ID: WP_107667006.1). In the case where the parent cyanobacterium is Spirulina, the gene coding the cell wall-pyruvic acid modification enzyme 9 may be, for example, csaB (NCBI Access ID: WP_026079530.1). In the case where the parent cyanobacterium is Calothrix, the gene coding the cell wall-pyruvic acid modification enzyme 9 may be, for example, csaB (NCBI Access ID: WP_096658142.1). In the case where the parent cyanobacterium is Nostoc, the gene coding the cell wall-pyruvic acid modification enzyme 9 may be, for example, csaB (NCBI Access ID: WP_099068528.1). In the case where the parent cyanobacterium is Crocosphaera, the gene coding the cell wall-pyruvic acid modification enzyme 9 may be, for example, csaB (NCBI Access ID: WP_012361697.1). In the case where the parent cyanobacterium is Pleurocapsa, the gene coding the cell wall-pyruvic acid modification enzyme 9 may be, for example, csaB (NCBI Access ID: WP_036798735). The base sequences of the above genes are available from the above-described NCBI database or Cyanobase.

Specifically, the gene coding the cell wall-pyruvic acid modification enzyme 9 may be, for example, slr0688 (SEQ ID NO: 10) of Synechocystis sp. PCC 6803, synpcc7942_1529 (SEQ ID NO: 11) of Synechococcus sp. PCC 7942, or anacy_1623 (SEQ ID NO: 12) of Anabaena cylindrica PCC 7122. A gene the base sequence of which is 50% or more identical to that of any of the above genes may also be used.

Thus, in the modified cyanobacterium, the gene coding any of the cell wall-pyruvic acid modification enzymes 9 represented by SEQ ID NOs: 10 to 12 or a gene the base sequence of which is 50% or more identical to that of any of the genes coding the above enzymes is deleted or inactivated. Therefore, in the modified cyanobacterium, (i) the expression of any of the above cell wall-pyruvic acid modification enzymes 9 or a protein having a function equivalent to that of any of the above enzymes is suppressed, or (ii) the function of any of the above cell wall-pyruvic acid modification enzymes 9 or a protein having a function equivalent to that of any of the above enzymes is suppressed. This reduces the likelihood of the covalently bonded sugar chains 3 present on the surface of the cell wall 4 being modified with pyruvic acid. Accordingly, the amount of bonds and bonding power between the SLH domain 7 of the SLH domain-containing outer membrane protein 6 included in the outer membrane 5 and the sugar chains 3 of the cell wall 4 are reduced. Thus, in the modified cyanobacterium according to the embodiment, the amount of the sugar chains 3, which are responsible for the bonding between the cell wall 4 and the outer membrane 5, modified with the pyruvic acid is reduced. This reduces the bonding power between the cell wall 4 the and outer membrane 5 and increases the likelihood of the outer membrane 5 partially detaching from the cell wall 4.

As described above, it is considered that proteins having substantially the same function are highly likely to be expressed when the base sequences of the genes coding the proteins are 30% or more identical to each other. Therefore, the gene coding the cell wall-pyruvic acid modification enzyme 9 the function of which is suppressed may be, for example, a gene that is composed of a base sequence that is 40% or more, preferably 50% or more, more preferably 60% or more, further preferably 70% or more, still more preferably 80% or more, and still further preferably 90% or more identical to the base sequence of any of the genes coding the cell wall-pyruvic acid modification enzymes 9 represented by SEQ ID NOs: 10 to 12 above and that codes a protein or polypeptide capable of catalyzing the modification reaction of the covalently bonded sugar chains 3 present in the peptidoglycan 2 of the cell wall 4 with pyruvic acid.

[6. Method for Preparing Modified Cyanobacterium]

A method for preparing the modified cyanobacterium according to the embodiment is described below. The method for preparing the modified cyanobacterium includes a step of suppressing the function of a protein responsible for the bonding between the outer membrane 5 and the cell wall 4 in a cyanobacterium.

In the present embodiment, the protein responsible for the bonding between the outer membrane 5 and the cell wall 4 may be, for example, at least one of the SLH domain-containing outer membrane protein 6 or the cell wall-pyruvic acid modification enzyme 9.

The method for suppressing the protein function is not limited. The above method may be, for example, deleting or inactivating the gene coding the SLH domain-containing outer membrane protein 6 and the gene coding the cell wall-pyruvic acid modification enzyme 9, inhibiting the transcription of the above genes, inhibiting the translation of the transcriptional products of the genes, or administering an inhibitor that specifically inhibits the above proteins.

The method for deleting or inactivating the genes may be, for example, introducing a mutation to one or more bases on the base sequences of the genes, replacing the above base sequences with other base sequences or inserting other base sequences to the above base sequences, or deleting a part or the entirety of the base sequences of the above genes.

The method for inhibiting the transcription of the above genes may be, for example, introducing a mutation to the promotor regions of the genes, inactivating the promotor by replacement with another base sequence or insertion of another base sequence, or CRISPR interference (NPL 13: Yao et al., ACS Synth. Biol., 2016, 5:207-212). Examples of the specific methods that may be used for performing the mutagenesis or the replacement or insertion of base sequences described above include ultraviolet irradiation, site directed mutagenesis, and homologous recombination.

The method for inhibiting the translation of the transcriptional products of the genes may be, for example, RNA (ribonucleic acid) interference.

For preparing the modified cyanobacterium, the function of the protein responsible for the bonding between the outer membrane 5 and the cell wall 4 in the cyanobacterium may be suppressed using any of the above methods.

Thus, in a modified cyanobacterium prepared using the above preparation method, the degree of bonding (i.e.,

amount of bonds and bonding power) between the cell wall 4 and the outer membrane 5 is partially reduced and, accordingly, the outer membrane 5 is likely to partially detach from the cell wall 4. Therefore, in the modified cyanobacterium, the proteins produced inside the bacterial cells are likely to leak out of the outer membrane 5 (i.e., out of the bacterial cells). Thus, the method for preparing the modified cyanobacterium according to the embodiment enables a modified cyanobacterium in which the secretory productivity of proteins is enhanced to be provided.

Moreover, since the proteins produced inside the bacterial cells leak out of the bacterial cells in the modified cyanobacterium prepared using the above preparation method, it is not necessary to crush the bacterial cells for collecting the proteins. For example, the modified cyanobacterium is cultivated under appropriate conditions, and the proteins secreted into the culture solution are subsequently collected. Thus, it is possible to collect the proteins present in the culture solution while cultivating the modified cyanobacterium. Therefore, the use of the modified cyanobacterium prepared using the above preparation method enables an efficient microbiological protein production to be achieved. Thus, the method for preparing the modified cyanobacterium according to the embodiment enables modified cyanobacteria having a high utilization efficiency, which can be repeatedly used after the proteins have been collected, to be provided.

Note that, in the modified cyanobacterium prepared using the above preparation method, a group of proteins that are naturally present mainly in the periplasm, such as peptidase and phosphatase, are secreted out of the cells. In the present embodiment, for example, it is possible to cause the modified cyanobacterium to produce an intended protein by modifying and replacing the gene coding a protein naturally produced inside a cyanobacterial cell, such as a group of proteins present in the periplasm, with a gene coding another protein. Thus, the method for preparing the modified cyanobacterium according to the embodiment also enables a modified cyanobacterium that produces an intended protein in an easy and simple manner and with efficiency to be provided.

[7. Method for Producing Protein Using Modified Cyanobacterium]

A method for producing a protein using the modified cyanobacterium according to the embodiment is described below. The method for producing a protein using the modified cyanobacterium according to the embodiment includes a step of cultivating the above-described modified cyanobacterium.

Cyanobacteria can be commonly cultivated by liquid culture using a BG-11 culture medium (see Table 2) or on the basis of a modification method thereof. Therefore, the modified cyanobacterium may be cultivated in the same manner as above. The culture period during which cyanobacteria are cultivated to produce proteins may be any period of time necessary for accumulating the proteins at a high concentration under the conditions where the bacterial cells are proliferated to a sufficient degree. The culture period may be, for example, 1 to 3 days and may also be 4 to 7 days. The culture method may be, for example, ventilated spinner culture or shake culture.

When the cultivation is performed under the above conditions, the modified cyanobacterium produces proteins inside the bacterial cells and secretes the proteins into the culture solution. When the proteins secreted in the culture solution are collected, the culture solution may be subjected to filtration, centrifugation, or the like in order to remove the solid component, such as cells (i.e., "bacterial cells"), from the culture solution and collect the culture supernatant. In the method for producing proteins using the modified cyanobacterium according to the embodiment, since the proteins are secreted out of the cells of the modified cyanobacterium, it is not necessary to crush the cells for collecting the proteins. Therefore, modified cyanobacteria that remain after the proteins have been collected can be repeatedly used to produce proteins.

The method for collecting proteins secreted in the culture solution is not limited to the above-described examples; the proteins present in the culture solution may be collected while the modified cyanobacteria are cultivated. For example, a permeable membrane permeable to proteins may be used and proteins that pass through the permeable membrane may be collected. In this case, effective microorganisms, such as lactic acid bacteria, may be cultivated using the proteins that pass through the permeable membrane as nutrient sources. Thus, the proteins present in the culture solution can be collected while the modified cyanobacterium is cultivated. This eliminates the need for a treatment that removes the bacterial cells of the modified cyanobacterium from the culture solution. Therefore, it is possible to produce a protein in a further easy and simple manner and with further efficiency.

In addition, it becomes unnecessary to collect bacterial cells from the culture solution and crush bacterial cells. This reduces damage and stress to the modified cyanobacterium. As a result, the secretory protein productivity of the modified cyanobacterium is unlikely to decrease, and the service life of the modified cyanobacterium can be increased.

As described above, the method for producing proteins using the modified cyanobacterium according to the embodiment enables, for example, enzymes used in the production of food ingredient raw materials and compounds, enzymes used for diagnosis or therapy in the medical field, enzymes used for livestock foods in the agricultural, fishery, and husbandry fields to be produced in an easy and simple manner and with efficiency.

[Examples]

The method for determining the deprivation of the outer membrane of a cyanobacterium according to the present disclosure and the apparatus for determining the deprivation of the outer membrane of a cyanobacterium according to the present disclosure are described specifically with reference to Examples below. It should be noted that the present disclosure is not limited only to Examples below.

In Examples below, in order to cause the outer membrane of a cyanobacterium to partially detach from the cell wall, the expression of the slr1841 gene coding the SLH domain-containing outer membrane protein was suppressed (Example 1), and the expression of the slr0688 gene coding the cell wall-pyruvic acid modification enzyme was suppressed (Example 2). That is, two types of modified cyanobacteria were prepared. The secretory protein productivity of each of the modified cyanobacteria was measured. Furthermore, the secreted proteins were identified. Note that the type of cyanobacterium used in Examples is Synechocystis sp. PCC 6803 (hereinafter, referred to simply as "cyanobacterium").

(Example 1)

In Example 1, a modified cyanobacterium in which the expression of the slr1841 gene coding the SLH domain-containing outer membrane protein was suppressed was prepared.

(1) Construction of Modified Cyanobacterial Strain in Which Expression of slr1841 Gene Was Suppressed

CRISPR (clustered regularly interspaced short palindromic repeat) interference was used for suppressing gene expression. In this method, the expression of the slr1841 gene can be suppressed by introducing a gene coding a dCas9 protein (hereinafter, referred to as "dCas9 gene") and slr1841_sgRNA (single-guide ribonucleic acid) gene into the chromosome DNA of a cyanobacterium. Moreover, the degree to which the expression of the slr1841 gene is suppressed can be controlled by controlling the transcription activity of the slr1841_sgRNA.

The mechanisms by which the gene expression is suppressed in this method are as follows.

A Cas9 protein (dCas9) the nuclease activity of which has been lost and sgRNA (slr1841_sgRNA) that complementarily binds to the base sequence of the slr1841 gene form a complex.

The complex recognizes the slr1841 gene present on the chromosome DNA of a cyanobacterium and specifically binds to the slr1841 gene. This bonding causes steric hindrance to inhibit the transcription of the slr1841 gene. As a result, the expression of the slr1841 gene of the cyanobacterium is suppressed. Moreover, the degree to which the expression of the slr1841 gene is suppressed can be controlled by controlling the transcription activity of the slr1841_sgRNA.

The method for introducing the two genes into the chromosome DNA of a cyanobacterium is described specifically below.

(1-1) Introduction of dCas9 Gene

Using the chromosome DNA of a Synechocystis LY07 strain (hereinafter, referred to also as "LY07 strain") (see NPL 13) as a template, a dCas9 gene, an operator gene used for controlling the expression of the dCas9 gene, and a spectinomycin resistance marker gene that served as a mark for gene introduction were amplified by a PCR (polymerase chain reaction) method using the primers psbA1-Fw (SEQ ID NO: 13) and psbA1-Rv (SEQ ID NO: 14) described in Table 1. Note that, since the three genes were inserted in the psbA1 gene of the chromosome DNA while being connected to one another in the LY07 strain, they can be amplified by a PCR method as a single DNA fragment. The resulting DNA fragment is referred to as "psbA1::dCas9 cassette". Using In-Fusion PCR cloning (registered trademark), the psbA1::dCas9 cassette was inserted into a pUC19 plasmid to prepare a pUC19-dCas9 plasmid.

[Table 1]

| Primer name | Base sequence | SEQ ID NO |
|---|---|---|
| psbA1-Fw | 5' -CAGTGAATTCGAGCTCGGTATATAGCGTTGCAGTCCCTGG-3' | 13 |
| psbA1-Rv | 5' -GATTACGCCAAGCTTGCATGACCGCGGTCACTTCATAACC-3' | 14 |
| slr2030-Fw | 5' -CAGTGAATTCGAGCTCGGTAATAACCGTTGTCCCTTTTGTTTCATCG-3' | 15 |
| sgRNA_ slr1841-Rv | 5' -TGTTAGTGAGCCCTGCTGTTAGCTCCCAGTATCTCTATCACTGAT-3' | 16 |

(continued)

| Primer name | Base sequence | SEQ ID NO |
|---|---|---|
| sgRNA_ slr1841-Fw | 5' -ACAGCAGGGCTCACTAACAGTTTTAGAGCTAGAAATAGCAAGTTAAAATAA-3' | 17 |
| slr2031-Rv | 5' -GATTACGCCAAGCTTGCATGGGGAACAAGCTGAATCTGGGCATC-3' | 18 |
| sgRNA_ slr0688-Rv | 5' -TTTTAGTCTGTTTGCTGCATAGCTCCCAGTATCTCTATCACTGAT-3' | 19 |
| sgRNA_ slr0688-Fw | 5' -TGCAGCAAACAGACTAAAAGTTTTAGAGCTAGAAATAGCAAGTTAAAATAA-3' | 20 |

With 1 μg of the thus-prepared pUC19-dCas9 plasmid, a culture solution of cyanobacteria (concentration of bacterial cells, OD730: about 0.5) was mixed, and the pUC19-dCas9 plasmid was introduced into the cyanobacterial cells by spontaneous transformation. The transformed cells were grown on a BG-11 agar culture medium containing 20 μg/mL spectinomycin to perform selection. In the selected cells, homologous recombination occurred between the psbA1 gene of the chromosome DNA and the psbA1 upstream and downstream fragment regions of the pUC19-dCas9 plasmid. Thus, a Synechocystis dCas9 strain having a psbA1 gene region in which the dCas9 cassette was inserted was prepared. Table 2 lists the composition of the BG-11 culture medium used.

[Table 2]

| Component | Content (mg/L) |
|---|---|
| EDTA-Na | 1 |
| Ammonium Iron Citrate | 6 |
| $NaNO_3$ | 1500 |
| $MgSO_4$ | 75 |
| $K_2HPO_4$ | 39 |
| $CaCl_2$ | 28.6 |
| $H_3BO_4$ | 2.8 |
| $MnCl_2$ | 1.8 |
| $ZnSO_4$ | 0.2 |
| $CuSO_4$ | 0.08 |
| $Na_2MoO_4$ | 0.02 |
| $Co(NO_3)_2$ | 0.005 |
| TES-KOH (pH 7.5) | 4580 |

(1-2) Introduction of slr1841_sgRNA Gene

In CRISPR interference, a sequence of about 20 bases which is complementary to the target sequence is introduced to a region of the sgRNA gene which is referred to as "protospacer" in order to cause the sgRNA to specifically binds to the target gene. Table 3 lists the protospacer sequences used in Examples.

[Table 3]

| Protospacer target gene | Base sequence | SEQ ID NO |
|---|---|---|
| slr1841 | 5' -ACAGCAGGGCTCACTAACA-3' | 21 |
| slr0688 | 5' -TGCAGCAAACAGACTAAAA-3' | 22 |

In Synechocystis LY04 strain, LY05 strain, or LY07 strain, the sgRNA gene (except the protospacer region) and the kanamycin resistance marker gene are inserted in the slr2030-slr2031 gene of the chromosome DNA while being connected to each other (see NPL 13). Therefore, a sgRNA that specifically recognizes slr1841 (slr1841_sgRNA) can be readily prepared by imparting a protospacer sequence (SEQ ID NO: 21) complementary to the slr1841 gene (SEQ ID NO: 7) into a primer used for amplifying the sgRNA gene by a PCR method. Moreover, the degree to which the expression of the slr1841 gene is suppressed can be controlled by controlling the transcription activity of the slr1841_sgRNA.

Using the chromosome DNA of the LY07 strain as a template, two DNA fragments were amplified by a PCR method with the set of the primer slr2030-Fw (SEQ ID NO: 15) and the sgRNA_slr1841-Rv (SEQ ID NO: 16) and the set of the sgRNA_slr1841-Fw (SEQ ID NO: 17) and the slr2031-Rv (SEQ ID NO: 18) described in Table 1.

Using a mixed solution of the above DNA fragments as a template, amplification was performed by a PCR method with the primers slr2030-Fw (SEQ ID NO: 15) and slr2031-Rv (SEQ ID NO: 18) described in Table 1 to prepare a DNA fragment (slr2030-2031::slr1841_sgRNA) including (i) the slr2030 gene fragment, (ii) slr1841_sgRNA, (iii) the kanamycin resistance marker gene, and (iv) the slr2031 gene fragment that were connected to one another in this order. The slr2030-2031::slr1841_sgRNA was inserted into a pUC19 plasmid using In-Fusion PCR cloning (registered trademark). Hereby, a pUC19-slr1841_sgRNA plasmid was prepared.

The pUC19-slr1841_sgRNA plasmid was introduced into the Synechocystis dCas9 strain, and transformed cells were selected on a BG-11 agar culture medium containing 30 μg/mL kanamycin, by the same method as in (1-1) above. Hereby, a Synechocystis dCas9 slr1841_sgRNA strain (hereinafter, referred to also as "slr1841 suppressed strain"), which was a transformant including the slr1841_sgRNA inserted in the slr2030-slr2031 gene of the chromosome DNA, was prepared.

(1-3) Suppression of slr1841 Gene

The promotor sequences of the dCas9 gene and the slr1841_sgRNA gene are designed such that expression induction occurs in the presence of anhydrotetracycline (aTc). In Examples, the expression of the slr1841 gene was suppressed by adding aTc to the culture medium at a final concentration of 1 μg/mL.

As described above, in Example 1, a modified cyanobacterium, Synechocystis dCas9 slr1841_sgRNA strain (i.e., "slr1841 suppressed strain"), in which the total amount of proteins responsible for the bonding between the outer membrane and the cell wall in a cyanobacterium was reduced to about 30% of the amount of proteins included in the parent strain (the Synechocystis dCas9 strain, Comparative Example 1 below), was prepared without impairing the proliferating ability of the cells. In the above example, the proteins responsible for the bonding between the outer membrane and the cell wall were slr1841, slr1908, and slr0042.

(Example 2)

In Example 2, a modified cyanobacterium in which the expression of the slr0688 gene coding the cell wall-pyruvic acid modification enzyme was suppressed was prepared in the following manner.

(2) Construction of Modified Cyanobacterial Strain in Which Expression of slr0688 Gene Was Suppressed

A sgRNA gene including a protospacer sequence (SEQ ID NO: 22) complementary to the slr0688 gene (SEQ ID NO: 4) was introduced into the Synechocystis dCas9 strain in the same manner as in (1-2) above to prepare a Synechocystis dCas9 slr0688_sgRNA strain. The above operation was done under the same conditions as in (1-2) above, except that the set of the primers slr2030-Fw (SEQ ID NO: 15) and sgRNA_slr0688-Rv (SEQ ID NO: 19) and the set of primers sgRNA_slr0688-Fw (SEQ ID NO: 20) and slr2031-Rv (SEQ ID NO: 18) described in Table 1 were used and that a DNA fragment (slr2030-2031::slr0688_sgRNA), which included (i) a slr2030 gene fragment, (ii) slr0688_sgRNA, (iii) a kanamycin resistance marker gene, and (iv) a slr2031 gene fragment connected to one another in this order, was inserted into a pUC19 plasmid using In-Fusion PCR cloning (registered trademark) to prepare a pUC19-slr0688_sgRNA plasmid. The degree to which the expression of the slr0688 gene is suppressed can be controlled by controlling the transcription activity of the slr0688_sgRNA.

The expression of the slr0688 gene was suppressed in the same manner as in (1-3) above.

As described above, in Example 2, a modified cyanobacterium, Synechocystis dCas9 slr0688_sgRNA strain (referred to also as "slr0688 suppressed strain"), in which the amount of proteins responsible for the bonding between the outer membrane and the cell wall in a cyanobacterium was reduced to about 50% of the amount of proteins included in the parent strain (the Synechocystis dCas9 strain, Comparative Example 1 below), was prepared without impairing the proliferating ability of the cells. In the above example, the protein responsible for the bonding between the outer membrane and the cell wall was slr0688.

(Comparative Example 1)

In Comparative Example 1, a Synechocystis dCas9 strain was prepared in the same manner as in (1-1) of Example 1.

The conditions of the surface layer of cells of each of the bacterial strains prepared in Example 1, Example 2, and Comparative Example 1 were inspected. The bacterial strains were also subjected to a protein secretory productivity test. Details of the inspection and test are described below.

(3) Inspection of Conditions of Surface Layer of Cells of Bacterial Strain

Ultrathin slices of the modified cyanobacterium, Synechocystis dCas9 slr1841_sgRNA strain (i.e., "slr1841 suppressed strain") prepared in Example 1, the modified cyanobacterium, Synechocystis dCas9 slr0688_sgRNA strain (i.e., "slr0688 suppressed strain") prepared in Example 2, and the modified cyanobacterium, Synechocystis dCas9 strain (hereinafter, referred to as "control strain") prepared in Comparative Example 1 were prepared. The conditions of the cell surface layers (i.e., the structures of outer membranes) were inspected with an electron microscope.

(3-1) Cultivation of Bacterial Strain

With the slr1841 suppressed strain prepared in Example 1, 12 mL of a BG-11 culture medium (see Table 2) including 1 μg/mL aTc was inoculated such that the initial concentration of the bacterial cells, OD730, was 0.05. Subsequently, shake culture was performed for 5 days at a photon flux density of 100 $\mu mol/m^2/s$ and 30°C. The slr0688 suppressed strain prepared in Example 2 and the control strain prepared in Comparative Example 1 were also cultivated under the same conditions as in Example 1.

(3-2) Preparation of Ultrathin Slice of Bacterial Strain

The culture solution prepared in (3-1) was centrifuged at 2,500 g for 10 minutes at room temperature in order to collect the cells of the slr1841 suppressed strain prepared in Example 1. Then, the cells were quick-frozen with liquid propane having a temperature of -175°C. Subsequently, immobilization was performed at -80°C for 2 days using an ethanol solution containing 2% glutaraldehyde and 1% tannic acid. The immobilized cells were dehydrated with ethanol. The dehydrated cells were impregnated with propylene oxide and subsequently immersed in a resin solution (Quetol-651). The solution was then left to stand at 60°C for 48 hours in order to cure the resin such that the cells were buried in the resin. The cells included in the resin were sliced into a thin piece having a thickness of 70 nm with an ultramicrotome (Ultracut) to prepare an ultrathin slice. The ultrathin slice was stained with a 2% uranium acetate-1% lead citrate solution to form a transmission electron microscope sample of the slr1841 suppressed strain prepared in Example 1. Note that the slr0688 suppressed strain prepared in Example 2 and the control strain prepared in Comparative Example 1 were also subjected to the same operation as described above in order to prepare transmission electron microscope samples.

(3-3) Electron Microscope Inspection

The ultrathin slices prepared in (3-2) were inspected using a transmission electron microscope (JEOL JEM-1400Plus) at an acceleration voltage of 100 kV. Figs. 4 to 9 illustrate the inspection results.

The slr1841 suppressed strain prepared in Example 1 is described below. Fig. 4 is a TEM (transmission electron microscope) image of the slr1841 suppressed strain prepared in Example 1. Fig. 5 is a magnified view of the broken-line region A in Fig. 4. Fig. 5(a) is a magnified TEM image of the broken-line region A in Fig. 4, while Fig. 5(b) is a diagram illustrating the magnified TEM image of Fig. 5(a).

As illustrated in Fig. 3, in the slr1841 suppressed strain prepared in Example 1, the outer membrane was partially deprived from the cell wall (i.e., the outer membrane is partially detached) and the outer membrane was partially warped.

The broken line region A was inspected using the magnified view in order to determine the conditions of the cell surface layer in further details. As illustrated in Figs. 5(a) and 5(b), portions in which the outer membrane was partially detached (the dot-and-dash line regions a1 and a2 in the drawing) were observed. Moreover, a portion in which the outer membrane was significantly warped was observed in the vicinity of the dot-and-dash line region a1. This portion is a portion in which the degree of bonding between the outer membrane and the cell wall was reduced. The present inventor believes that the culture solution penetrated the periplasm through the outer membrane and caused the outer membrane to be swollen outward, which made the outer membrane warped.

The slr0688 suppressed strain prepared in Example 2 is described below. Fig. 6 is a TEM image of the slr0688 suppressed strain prepared in Example 2. Fig. 7 is a magnified view of the broken-line region B in Fig. 6. Fig. 7(a) is a magnified TEM image of the broken-line region B in Fig. 6, while Fig. 7(b) is a diagram illustrating the magnified TEM image of Fig. 7(a).

As illustrated in Fig. 6, in the slr0688 suppressed strain prepared in Example 2, the outer membrane was partially deprived from the cell wall and the outer membrane was partially warped. Moreover, in the slr0688 suppressed strain, it was observed that the outer membrane was partially detached from the cell wall.

The broken line region B was inspected using the magnified view in order to determine the conditions of the cell surface layer in further details. As illustrated in Figs. 7(a) and 7(b), a portion in which the outer membrane was significantly warped (the dot-and-dash line region b1 in the drawing) and portions in which the outer membrane was partially detached (the dot-and-dash line regions b2 and b3 in the drawing) were observed. Moreover, a portion in which the outer membrane was detached from the cell wall was observed in the vicinity of each of the dot-and-dash line regions b1, b2, and b3.

The control strain prepared in Comparative Example 1 is described below. Fig. 8 is a TEM image of the control strain prepared in Comparative Example 1. Fig. 9 is a magnified view of the broken-line region C in Fig. 8. Fig. 9(a) is a magnified TEM image of the broken-line region C in Fig. 8, while Fig. 9(b) is a diagram illustrating the magnified TEM image of Fig. 9(a).

As illustrated in Figs. 8 and 9, the cell surface layer of the control strain prepared in Comparative Example 1 was kept in a regular manner and included the inner membrane, the cell wall, the outer membrane, and the S-layer stacked on top of one another in order. In other words, in the control strain, a portion in which the outer membrane was detached form the cell wall, a portion in which the outer membrane was deprived (i.e., peeled off) from the cell wall, and a portion in which the outer membrane was warped as in Examples 1 and 2 were not observed.

(4) Secretory Protein Productivity Test

The slr1841 suppressed strain prepared in Example 1, the slr0688 suppressed strain prepared in Example 2, and the control strain prepared in Comparative Example 1 were cultivated. The amounts of proteins secreted out of the cells (hereinafter, such an amount is referred to also as "amount of proteins secreted") were measured. The secretory protein productivity of each of the above bacterial strains was evaluated on the basis of the amount of proteins present in the culture solution. Note that the term "secretory protein productivity" used herein refers to an ability to produce proteins by secreting proteins produced inside the cells out of the cells. A specific method is described below.

(4-1) Cultivation of Bacterial Strain

The slr1841 suppressed strain prepared in Example 1 was cultivated as in (3-1). The cultivation was performed three times independently. The bacterial strains prepared in Example 2 and Comparative Example 1 were also cultivated under the same conditions as the bacterial strain prepared in Example 1.

(4-2) Quantification of Proteins Secreted out of Cells

The culture solution prepared in (4-1) was centrifuged at 2,500 g for 10 minutes at room temperature to form a culture supernatant. The culture supernatant was filtered through a membrane filter having a pore size of 0.22 $\mu$m in order to completely remove the cells of the slr1841 suppressed strain prepared in Example 1. The total amount of proteins included in the filtered culture supernatant was quantified by a BCA (bicinchoninic acid) method. Each of the three culture solutions cultivated individually was subjected to the series of procedures above, and the average amount of proteins secreted out of the cells of the slr1841 suppressed strain prepared in Example 1 and the standard deviation thereof were determined. The bacterial strains prepared in Example 2 and Comparative Example 1 were also subjected to the quantification of proteins included in the three culture solutions under the same conditions as described above, and the average of the amounts of proteins included in the three culture solutions and the standard deviation thereof were determined.

Fig. 10 illustrates the results. Fig. 10 includes graphs illustrating the mass of proteins in the culture supernatants of the modified cyanobacteria prepared in Example 1, Example 2, and Comparative Example 1 (n = 3, error bar: SD).

As illustrated in Fig. 10, in both of the slr1841 suppressed strain and the slr0688 suppressed strain prepared in Examples 1 and 2, the mass (mg/L) of proteins secreted into the culture supernatant was increased by about 25 times compared with the control strain prepared in Comparative Example 1.

Although the description of data is omitted, the mass of proteins secreted per dry weight of bacterial cells (mg protein/g cell dry weight) was calculated by measuring the absorbance (730 nm) of the culture solution. It was found that, in both of the slr1841 suppressed strain and the slr0688 suppressed strain prepared in Examples 1 and 2, the amount of proteins secreted per dry weight of bacterial cells (mg protein/g cell dry weight) was increased by about 36 times compared with the control strain prepared in Comparative Example 1.

As illustrated in Fig. 10, the mass of proteins secreted into the culture supernatant was larger in the slr0688 suppressed strain prepared in Example 2, in which the expression of the gene (slr0688) coding the cell wall-pyruvic acid modification enzyme was suppressed, than in the slr1841 suppressed strain prepared in Example 1, in which the expression of a gene (slr1841) coding the SLH domain-containing outer membrane protein was suppressed. The present inventor believes that this is because the number of molecules of the covalently bonded sugar chains present in the surface of the cell wall is larger than the number of molecules of the SLH domain-containing outer membrane protein (Slr1841) present in the outer membrane. In other words, the present inventor believes that, in the slr0688 suppressed strain prepared in Example 2, the amount of bonds and bonding power between the outer membrane and the cell wall were further reduced compared with the slr1841 suppressed strain prepared in Example 1 and, consequently, the mass of proteins secreted was larger than in the slr1841 suppressed strain prepared in Example 1.

The above results confirm that, when the function of proteins responsible for the bonding between the outer membrane and the cell wall is suppressed, the degree of bonding between the outer membrane and the cell wall in a cyanobacterium can be partially reduced and, consequently, the outer membrane may partially detach from the cell wall. It was also confirmed that, as a result of the reduction in the degree of bonding between the outer membrane and the cell wall, the likelihood of proteins produced inside the cells of the cyanobacterium leaking out of the cells is increased. Thus, it was confirmed that a modified cyanobacterium having a markedly improved secretory protein productivity is prepared using the modified cyanobacterium according to the embodiment and a method for preparing the modified cyanobacterium.

(5) Identification of Secreted Proteins

The secreted proteins included in the culture supernatant prepared in (4-2) were identified by LC-MS/MS. The method is described below.

(5-1) Sample Preparation

Cold acetone was added to the culture supernatant in an amount 8 times the amount of the culture supernatant. After the resulting mixture was left to stand at 20°C for 2 hours, it was centrifuged at 20,000 g for 15 minutes in order to form a protein precipitate. To the precipitate, 100 mM Tris pH 8.5 and 0.5% sodium dodecanoate (SDoD) were added. Subsequently, the proteins were dissolved with a closed ultrasonic crusher. After the concentration of the proteins had been adjusted to 1 μg/mL, dithiothreitol (DTT) was added to the solution at a final concentration of 10 mM. Then, the solution was left to stand at 50°C for 30 minutes. Subsequently, iodoacetamide (IAA) was added to the solution at a final concentration of 30 mM. The solution was then left to stand for 30 minutes at room temperature (lightproof). In order to stop the reaction of IAA, cysteine was added to the solution at a final concentration of 60 mM, and the solution was then left to stand for 10 minutes at room temperature. To the solution, 400 ng of trypsin was added, and the resulting solution was left to stand over a night at 37°C in order to form the proteins into peptide fragments. Subsequent to the addition of 5% TFA (trifluoroacetic acid), centrifugation was performed at 15,000 g for 10 minutes at room temperature. Hereby, a supernatant was prepared. Note that SDoD was removed by the above procedures. After desalination had been performed using C18 spin columns, the sample was dried with a centrifugal evaporator. Subsequently, 3% acetonitrile and 0.1% formic acid were added to the sample, and the sample was dissolved therein with a closed ultrasonic crusher. The peptide concentration was adjusted to 200 ng/μL.

(5-2) LC-MS/MS Analysis

The sample prepared in (5-1) was analyzed using an LC-MS/MS apparatus (UltiMate 3000 RSLCnano LC System) under the following conditions.

Volume of sample injected: 200 ng
Column: CAPCELL CORE MP 75 μm × 250 mm
Solvent: Solvent A was 0.1% aqueous formic acid solution, and Solvent B was 0.1% formic acid + 80% acetonitrile
Gradient program: 4 minutes after sample injection, Solvent B: 8%, 27 minutes after sample injection, Solvent B: 44%, 28 minutes after sample injection, Solvent B: 80%, and the measurement was finished 34 minutes after sample injection.

(5-3) Data Analysis

The data were analyzed under the following conditions in order to identify the proteins and peptides and calculate the quantitative values.

Software: Scaffold DIA
Database: UniProtKB/Swiss Prot database (Synechocystis sp. PCC 6803)

Fragmentation: HCD
Precursor Tolerance: 8 ppm
Fragment Tolerance: 10 ppm
Data Acquisition Type: Overlapping DIA
Peptide Length: 8 to 70
Peptide Charge: 2 to 8
Max Missed Cleavages: 1
Fixed Modification: Carbamidomethylation
Peptide FDR: 1% or less

Table 4 lists ten types of proteins the relative quantitative values of which were the largest among the identified proteins, in the order of largest value.

[Table 4]

| | Protein name | Uniprot Accession ID | Gene name |
|---|---|---|---|
| 1 | Carboxyl-terminal protease | P73458 | prc |
| 2 | Ssr1853 protein | P72639 | ssr1853 |
| 3 | Sll0314 protein | Q55648 | sll0314 |
| 4 | Sll0858 protein | P73742 | sll0858 |
| 5 | Sll0319 protein | P74789 | sll0319 |
| 6 | General secretion pathway protein G | P73704 | hofG |
| 7 | Slr0581 protein | Q55408 | slr0581 |
| 8 | Uncharacterized WD repeat-containing protein sll1491 | P74598 | sll1491 |
| 9 | Iron uptake protein A2 | Q55835 | futA2 |
| 10 | Plastocyanin | P21697 | petE |

The ten types of proteins above were all included in each of the culture supernatant of the slr1841 suppressed strain prepared in Example 1 and the culture supernatant of the slr0688 suppressed strain prepared in Example 2. All of the above proteins held a periplasm (refers to the gap between the outer membrane and the inner membrane) localization signal. The above results confirm that, in the modified strains prepared in Examples 1 and 2, the likelihood of the proteins present inside the periplasm leaking out of the outer membrane (i.e., out of the bacterial cells) was increased as a result of the outer membrane being partially detached from the cell wall. Thus, it was confirmed that the secretory protein productivity of the modified cyanobacterium according to the embodiment was markedly enhanced.

(6) Determination of Deprivation of Outer Membrane of Cyanobacterium

(6-1) Cultivation of Cyanobacterium

The control strain prepared in Comparative Example 1 was used as a wild-type strain of a cyanobacterium Synechocystis sp. PCC 6803. The slr0688 suppressed strain prepared in Example 2, which was confirmed that the outer membrane was deprived in (4) Secretory Protein Production Test, was used as an outer membrane-deprived Synechocystis sp. PCC 6803 mutant strain. Hereinafter, the above cyanobacteria are referred to as "cyanobacterium wild-type strain" and "outer membrane-deprived cyanobacterium", respectively. With the two types of cyanobacteria, 12 mL of a BG-11 culture medium (see Table 2) including 1 μg/mL aTc charged in a 125-mL Erlenmeyer flask was inoculated such that the initial concentration of the bacterial cells, OD730, was 0.1. Subsequently, shake culture was performed for 3 days at a photon flux density of 100 $\mu mol/m^2/s$ and 30°C.

The culture solutions that had been cultivated for 3 days were centrifuged at 2,500 g for 10 minutes at room temperature. The resulting culture supernatants were filtered through Millex-GV Syringe Filter Unit, 0.22 μm (Millipore). The filtered supernatants were subjected to an amino acid analysis by LC-MS/MS.

(6-2) Amino Acid Analysis by LC-MS/MS

The amounts of isoleucine, leucine, tyrosine, and phenylalanine included in the culture supernatants of the two types of cyanobacteria prepared in (6-1) were measured by LC-MS/MS. The method is described below.

(6-2-1) Sample Preparation

After 1 mL of the culture supernatant of the cyanobacterium wild-type strain prepared in (6-1) had been freeze-dried, it was dissolved in 195 μL of APDSTAG (registered trademark) Wako Borate Buffer. To the resulting solution, 5 μL of a solution prepared by dissolving 100 mg of an APDS (3-aminopyridyl-N-hydroxysuccinimidyl carbamate) tag reagent in a 5 mL of acetonitrile was added. The resulting mixture was stirred to a sufficient degree and subsequently heated at 55°C to 60°C for 5 to 15 minutes. The culture supernatant of the outer membrane-deprived cyanobacterium was also treated in the same manner as described above.

(6-2-2) LC-MS/MS Analysis

The sample prepared in (6-2-1) was analyzed using an LC-MS/MS apparatus (UltiMate 3000 RSLCnano LC System) under the following conditions.

Volume of sample injected: 10 μL
Column:

Tosoh TSKgel ODS-100V 2.0 mm I.D × 15 cm

Mobile phase: Solution A: APDSTAG (registered trademark) Wako eluant, Solution B: 60% acetonitrile
Elution time table (Solution B, %):

0 min, 6%; 0.05 to 1.70 min, 8%; 1.71 min, 12%; 4.95 min, 30%; 5.95 min, 60%; 6.70 min, 95%; 6.71 min, 6%; 12.0 min, 6%
Flow rate: 0.3 mL/min
Column temperature: 40°C
Ionization mode: ESI positive

(6-3) Results

Fig. 11 is a diagram illustrating the results of the amino acid analysis of the culture supernatants of the two types of cyanobacteria by LC-MS/MS. Fig. 11 illustrates the TICs (total ion chromatograms) of the culture supernatants of the outer membrane-deprived cyanobacterium and the cyanobacterium wild-type strain, where A, B, C, and D denote the peaks derived from tyrosine, isoleucine, leucine, and phenylalanine, respectively. As illustrated in Fig. 11, the peaks A to D were substantially not detected in the culture supernatant of the cyanobacterium wild-type strain, but detected in the culture supernatant of the outer membrane-deprived cyanobacterium. This confirms that, when a cyanobacterium in which the outer membrane has been deprived from the cell wall is cultivated, they are typically secreted into the culture supernatant.

Table 5 lists the results of measurement of the concentrations of the four amino acids. As listed in Table 5, isoleucine, leucine, tyrosine, and phenylalanine were present in the culture supernatant of the outer membrane-deprived cyanobacterium at concentrations of about 130 nM or more and 320 nM or less. In contrast, that the four amino acids were substantially absent in the culture supernatant of the cyanobacterium wild-type strain.

Thus, it was confirmed that whether the outer membrane is deprived from the cell wall can be determined in an easy and simple manner by quantifying at least one amino acid selected from the group consisting of the four amino acids included in the culture supernatant. On the basis of the results described in Table 5, the present inventor believes that, when the concentration of each of the amino acids is more than 100 nM, it can be determined that the outer membrane of the cyanobacteria has been deprived from the cell wall. The use of the method for determining deprivation of an outer membrane of a cyanobacterium according to the present disclosure eliminates the need to check the conditions of the outer membrane using a complex method, such as a method including collecting the cells of the cyanobacterium and inspecting them with an electron microscope. Therefore, it becomes possible to select cells suitable for matter production and use the cells for matter production quickly and in an easy and simple manner.

[Table 5]

| | Isoleucine (nM) | Leucine (nM) | Tyrosine (nM) | Phenylalanine (nM) |
|---|---|---|---|---|
| Outer membrane-deprived cyanobacterium | 171.2±39.1 | 160.8±32.1 | 313.9±111.3 | 212.9±87.5 |

(continued)

| | Isoleucine (nM) | Leucine (nM) | Tyrosine (nM) | Phenylalanine (nM) |
|---|---|---|---|---|
| Cyanobacterium wild-type strain | 17.9±21.3 | 20.1±19.7 | 28.7±20.3 | 15.7±19.4 |

(7) Discussions

In Examples, it was confirmed that the modified cyanobacterium according to the present disclosure secretes proteins present inside the bacterial cells (i.e., inside the periplasm) out of the bacterial cells. Since the modified cyanobacterium according to the present disclosure can be genetically modified so as to produce, for example, another protein instead of the proteins identified above (i.e., the proteins naturally produced inside the bacterial cells), it becomes possible to produce intended proteins with efficiency. In addition, since cyanobacteria have a high photosynthetic ability, it is possible to produce necessary proteins at necessary timings in an easy and simple manner by cultivating cyanobacteria with light, water, air, and a trace amount of inorganic substances. Thus, it is not necessary to use complex apparatuses for producing proteins. The function of a protein is likely to be lost when, for example, the protein is formed into a supplement or the like. Therefore, the modified cyanobacterium according to the present disclosure enables proteins to be provided while the functions of the proteins are maintained. Due to the above advantages, it is anticipated that the modified cyanobacterium according to the present disclosure can be applied to various fields.

In "(3-3) Electron Microscope Inspection", it was observed that, in the modified strain in which the expression of a gene coding a protein responsible for the bonding between the outer membrane and the cell wall was suppressed, the outer membrane was partially detached and the outer membrane was partially swollen due to the reduction in the degree of bonding between the outer membrane and the cell wall. The present inventor believes that this is because, with a reduction in the degree of bonding between the outer membrane and the cell wall, the likelihood of the culture solution entering the portion in which the outer membrane is partially detached from the cell wall from the outside of the cells due to the osmotic pressure is increased, and the culture solution causes the outer membrane to partially swell. Since the difference in protein concentration between the culture solution and the periplasm is large, the entry of the culture solution into the periplasm through the outer membrane does not stop. The present inventor believes that, when the outer membrane cannot withstand the internal pressure, it ruptures and becomes detached (i.e., deprived).

Table 3 lists only ten types of proteins the relative quantitative values of which were the largest among the proteins identified by LC-MS/MS analysis, in the order of largest value. Although it is described in "(5-3) Data Analysis" that all of the ten types of proteins described in Table 3 were included in each of the culture supernatants of the slr1841 suppressed strain prepared in Example 1 and the slr0688 strain prepared in Example 2, it was observed that all of the other proteins identified by the LC-MS/MS analysis were also included in each of the culture supernatants of Examples 1 and 2. It was observed that the secreted proteins included the proteins naturally present inside the periplasm and the proteins that are transported from the cytoplasm to the periplasm and function inside the periplasm.

Table 5 lists the concentrations of four amino acids typically included in the culture supernatant of the outer membrane-deprived cyanobacterium which were determined by LC-MS/MS analysis. The four amino acids were substantially absent in the culture supernatant of the cyanobacterium wild-type strain. This confirms that it is possible to determine that the outer membrane of the cyanobacterium is deprived from the cell wall when the concentration of any of the four amino acids in the culture supernatant is more than a threshold (e.g., 100 nM).

(Other Embodiments)

The method for determining deprivation of the outer membrane of a cyanobacterium, the apparatus for determining deprivation of the outer membrane of a cyanobacterium, and a program according to the present disclosure are described above on the basis of the embodiments. The present disclosure is not limited to the above embodiments. It should be noted that an embodiment constructed by applying various modifications that can be conceived by those skilled in the art to the above-described embodiments and an embodiment constructed by combining some elements of the above-described embodiments with one another are also within the scope of the present disclosure without departing from the summary of the present disclosure.

Although, in the example described in the above embodiment, the total amount of proteins responsible for the bonding between the outer membrane and the cell wall in a cyanobacterium is reduced to 30% or more and 70% or less of the total amount of the proteins included in the parent strain in order to reduce the degree of bonding between the outer membrane and the cell membrane and thereby cause the proteins produced inside the bacterial cells to leak out of the bacterial cells, the present disclosure is not limited thereto. Alternatively, the degree of bonding between the outer membrane and the cell wall may be reduced by, for example, applying external forces to the cyanobacterium. In another case, the outer membrane may be embrittled. The embrittlement of the outer membrane may also be achieved by adding an enzyme or chemical to the culture solution of the cyanobacterium.

Industrial Applicability

According to the present disclosure, whether the outer membrane of the cyanobacteria is deprived from the cell wall can be determined in an easy and simple manner. This makes it possible to quickly determine whether the conditions of cyanobacteria are suitable for matter production and whether the conditions of cyanobacteria are maintained suitable for matter production even in a cultivation process. Thus, according to the present disclosure, cyanobacteria the conditions of which are suitable for matter production can be selectively used. This increases matter productivity with certainty.

Reference Signs List

1 inner membrane
2 peptidoglycan
3 sugar chain
4 cell wall
5 outer membrane
6 SLH domain-containing outer membrane protein
7 SLH domain
8 organic substance channel protein
9 cell wall-pyruvic acid modification enzyme

**Claims**

**1.** A method for determining deprivation of an outer membrane of a cyanobacterium, the method comprising:

a measurement step in which a concentration of at least one amino acid selected from the group consisting of isoleucine, leucine, tyrosine, and phenylalanine in a culture supernatant of a cyanobacterium is measured; and
a determination step in which whether an outer membrane of the cyanobacterium is deprived from a cell wall of the cyanobacterium is determined on the basis of the concentration of the at least one amino acid, the concentration being measured in the measurement step.

**2.** The method for determining deprivation of the outer membrane of the cyanobacterium according to claim 1, wherein,

in the measurement step, concentrations of four amino acids of isoleucine, leucine, tyrosine, and phenylalanine are measured, and
in the determination step, whether the outer membrane of the cyanobacterium is deprived from the cell wall of the cyanobacterium is determined on the basis of the concentrations of the four amino acids.

**3.** The method for determining deprivation of the outer membrane of the cyanobacterium according to claim 1, wherein, in the determination step, it is determined that the outer membrane of the cyanobacterium is deprived from the cell wall when at least one of the concentration of the at least one amino acid is equal to or more than a threshold, and it is determined that the outer membrane of the cyanobacterium is not deprived from the cell wall when all of the concentration of the at least one amino acid is less than the threshold.

**4.** The method for determining deprivation of the outer membrane of the cyanobacterium according to claim 3, wherein the threshold is 100 nM.

**5.** An apparatus for determining deprivation of an outer membrane of a cyanobacterium, the apparatus comprising:

a measurement unit that measures a concentration of at least one amino acid selected from the group consisting of isoleucine, leucine, tyrosine, and phenylalanine in a culture supernatant of a cyanobacterium; and
a determination unit that determines whether the outer membrane of the cyanobacterium is deprived from a cell wall of the cyanobacterium on the basis of the concentration of the at least one amino acid, the concentration being measured by the measurement unit.

**6.** A program for causing a computer to execute a method for determining whether an outer membrane of a cyanobacterium is deprived from a cell wall of the cyanobacterium on the basis of a concentration of at least one amino acid selected from the group consisting of isoleucine, leucine, tyrosine, and phenylalanine in a culture supernatant of the cyanobacterium.

**7.** A method for determining deprivation of an outer membrane of a cyanobacterium, the method comprising:

a determination step in which whether an outer membrane of the cyanobacterium is deprived from a cell wall of the cyanobacterium is determined on the basis of a concentration of an amino acid in a culture supernatant of the cyanobacterium,
the amino acid being at least one amino acid selected from the group consisting of isoleucine, leucine, tyrosine, and phenylalanine.

FIG. 1

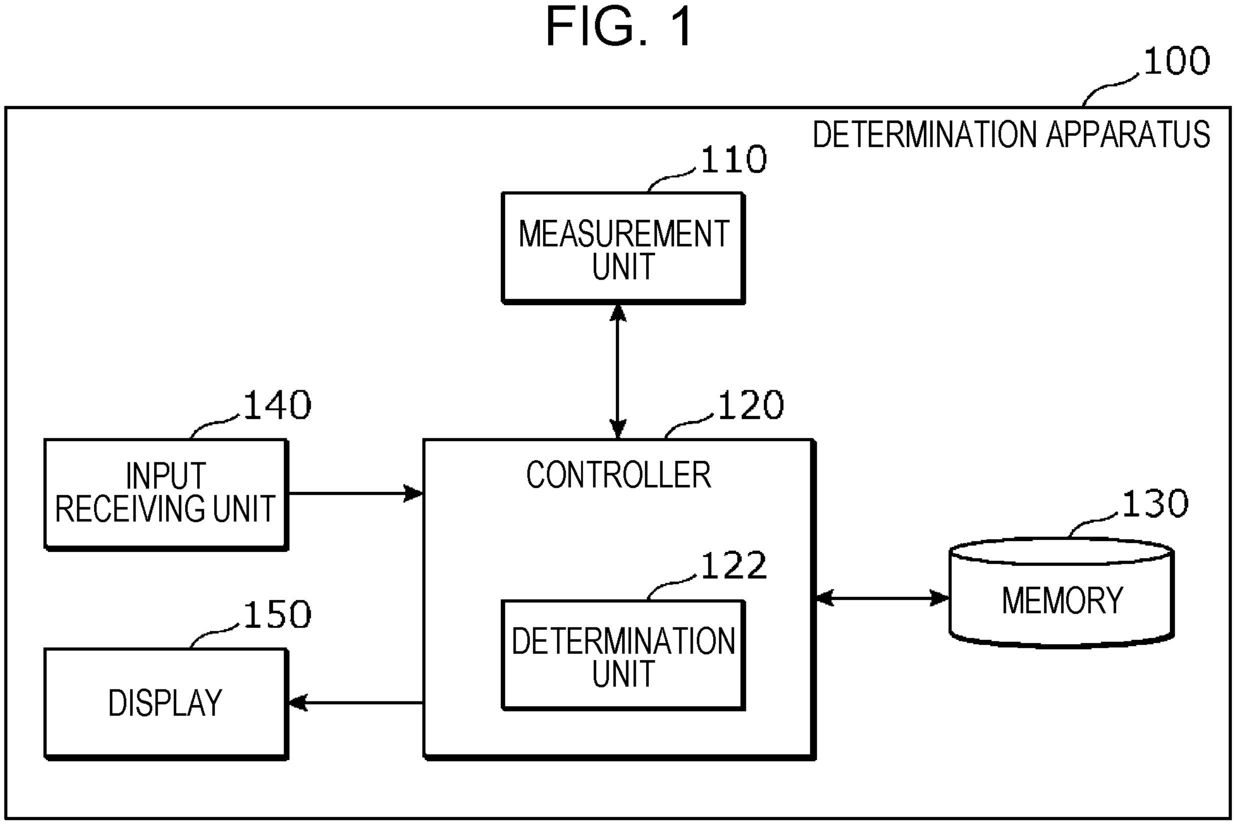
100
DETERMINATION APPARATUS
110
MEASUREMENT UNIT
140
INPUT RECEIVING UNIT
120
CONTROLLER
130
MEMORY
122
DETERMINATION UNIT
150
DISPLAY

FIG. 2A

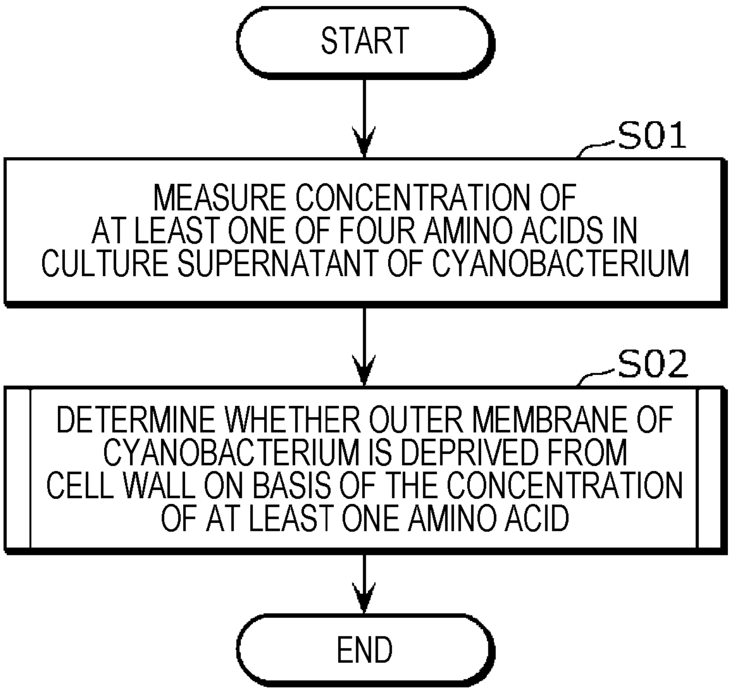
START
S01
MEASURE CONCENTRATION OF AT LEAST ONE OF FOUR AMINO ACIDS IN CULTURE SUPERNATANT OF CYANOBACTERIUM
S02
DETERMINE WHETHER OUTER MEMBRANE OF CYANOBACTERIUM IS DEPRIVED FROM CELL WALL ON BASIS OF THE CONCENTRATION OF AT LEAST ONE AMINO ACID
END

FIG. 2B

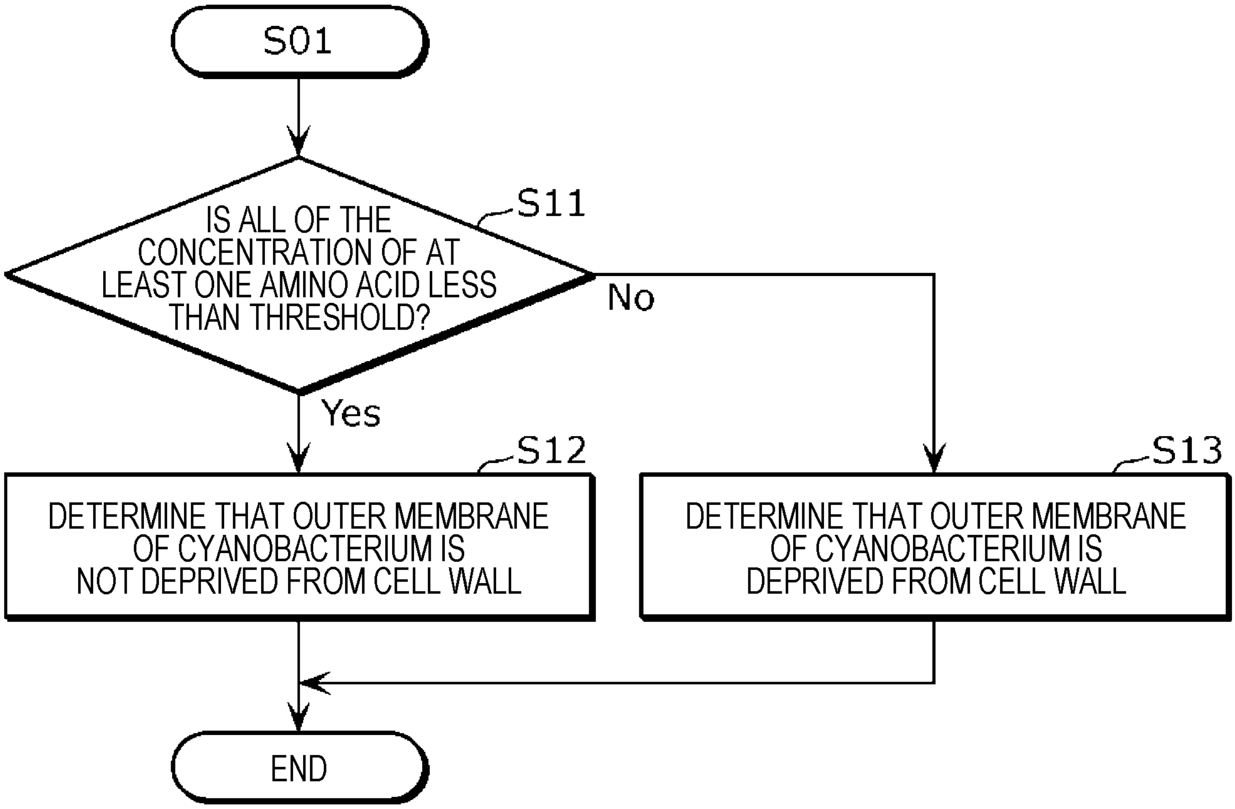
S01
IS ALL OF THE CONCENTRATION OF AT LEAST ONE AMINO ACID LESS THAN THRESHOLD?
S11
No
Yes
S12
DETERMINE THAT OUTER MEMBRANE OF CYANOBACTERIUM IS NOT DEPRIVED FROM CELL WALL
S13
DETERMINE THAT OUTER MEMBRANE OF CYANOBACTERIUM IS DEPRIVED FROM CELL WALL
END

FIG. 3

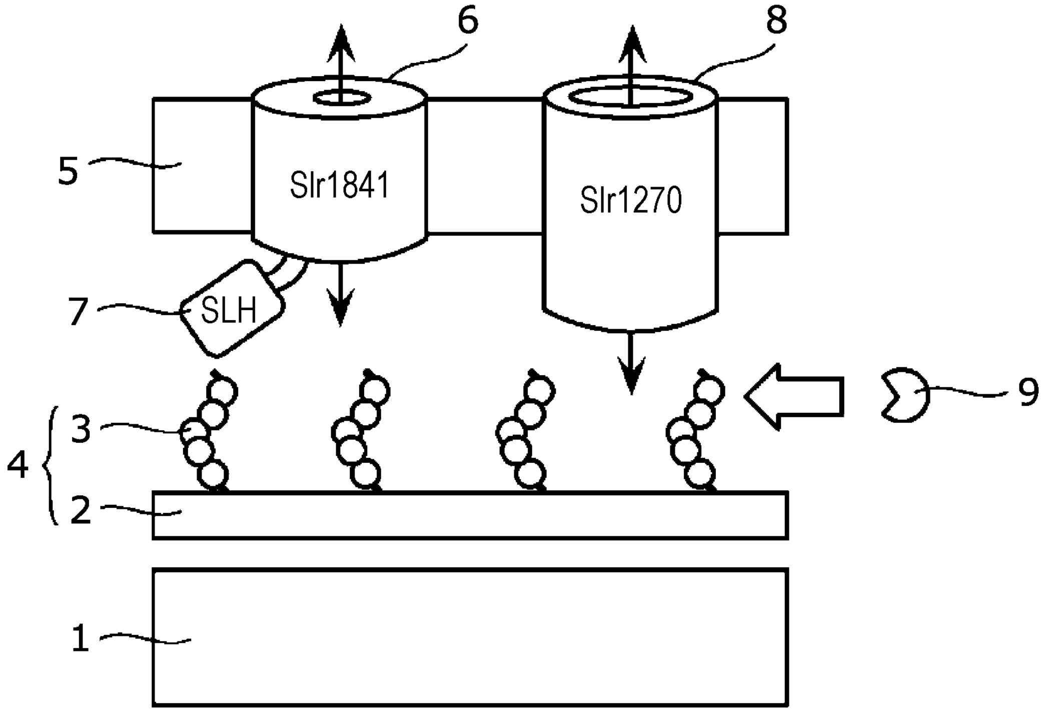
6
8
5
Slr1841
Slr1270
7
SLH
9
3
4
2
1

FIG. 4

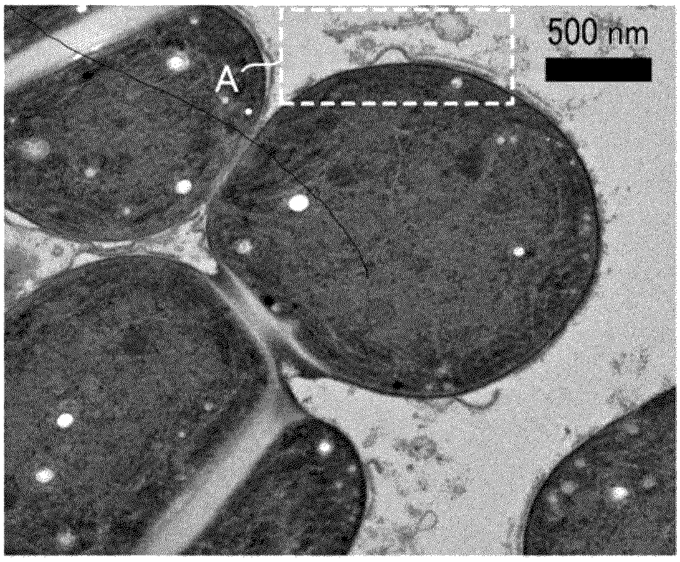
500 nm
A

FIG. 5

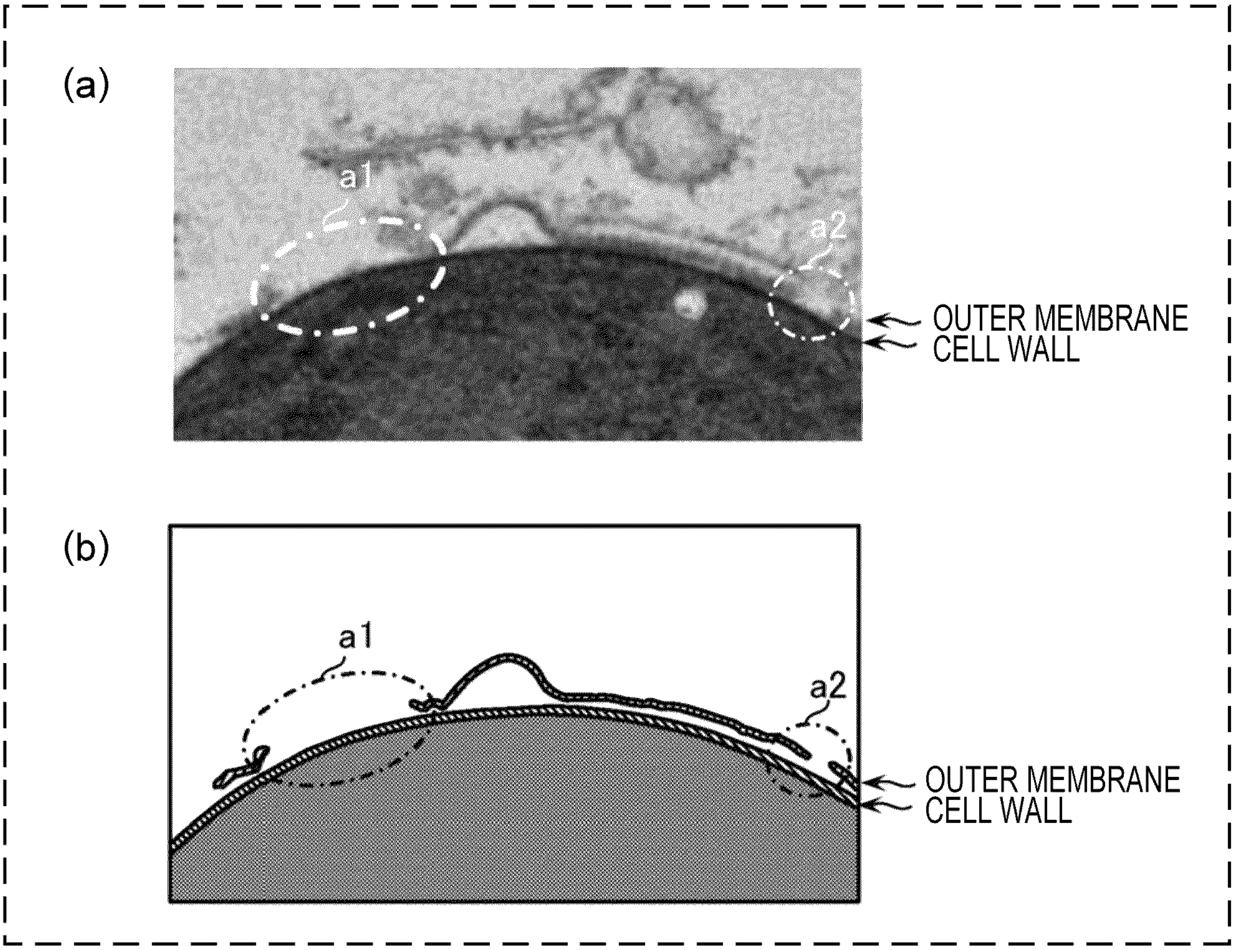
(a)
a1
a2
OUTER MEMBRANE
CELL WALL
(b)
a1
a2
OUTER MEMBRANE
CELL WALL

FIG. 6

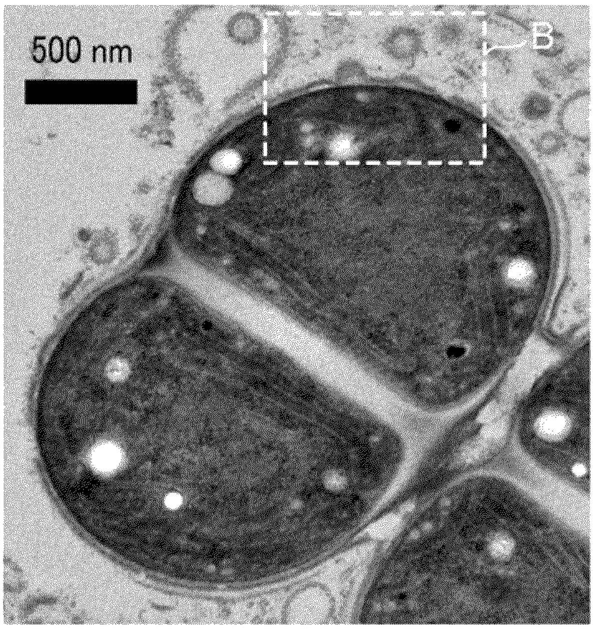
500 nm
B

FIG. 7

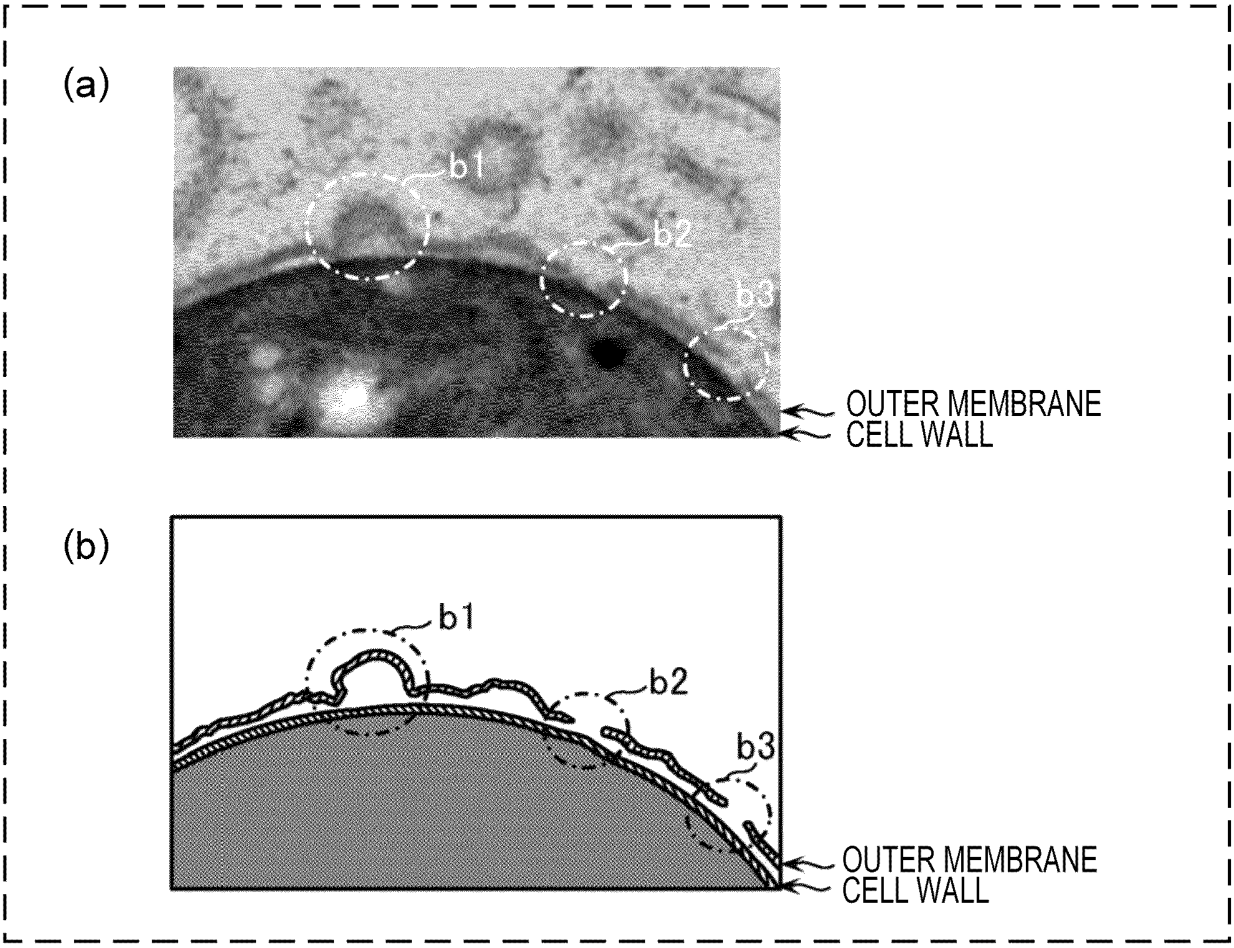
(a)
b1
b2
b3
OUTER MEMBRANE
CELL WALL
(b)
b1
b2
b3
OUTER MEMBRANE
CELL WALL

FIG. 8

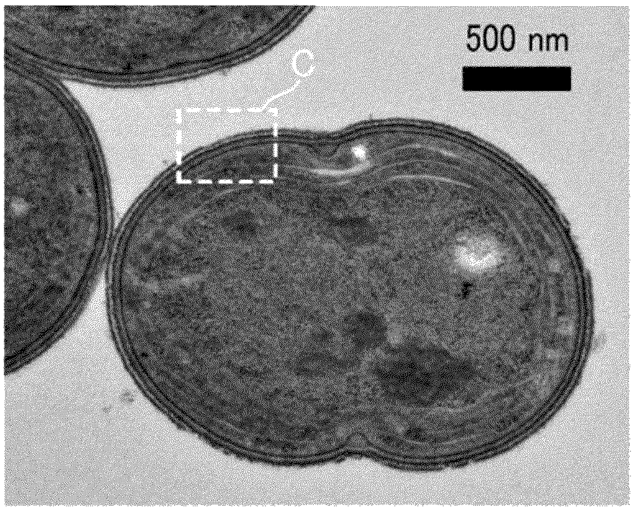


FIG. 9

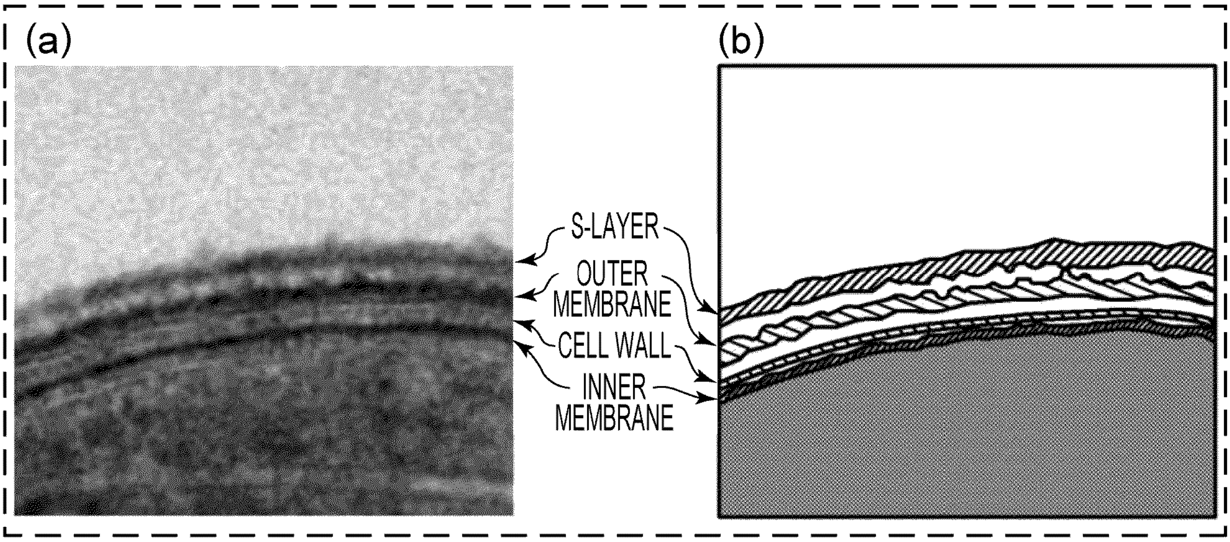


FIG. 10

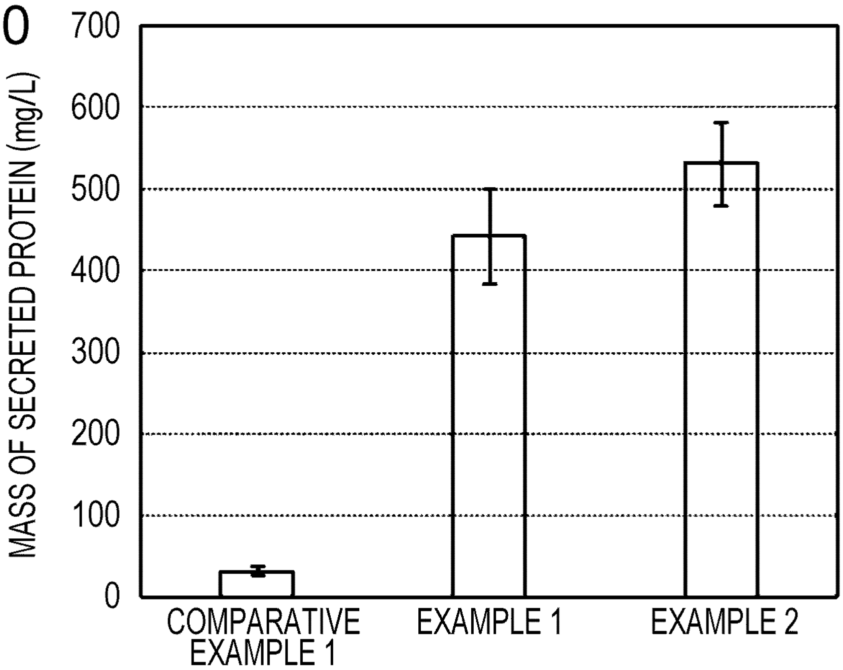

FIG. 11

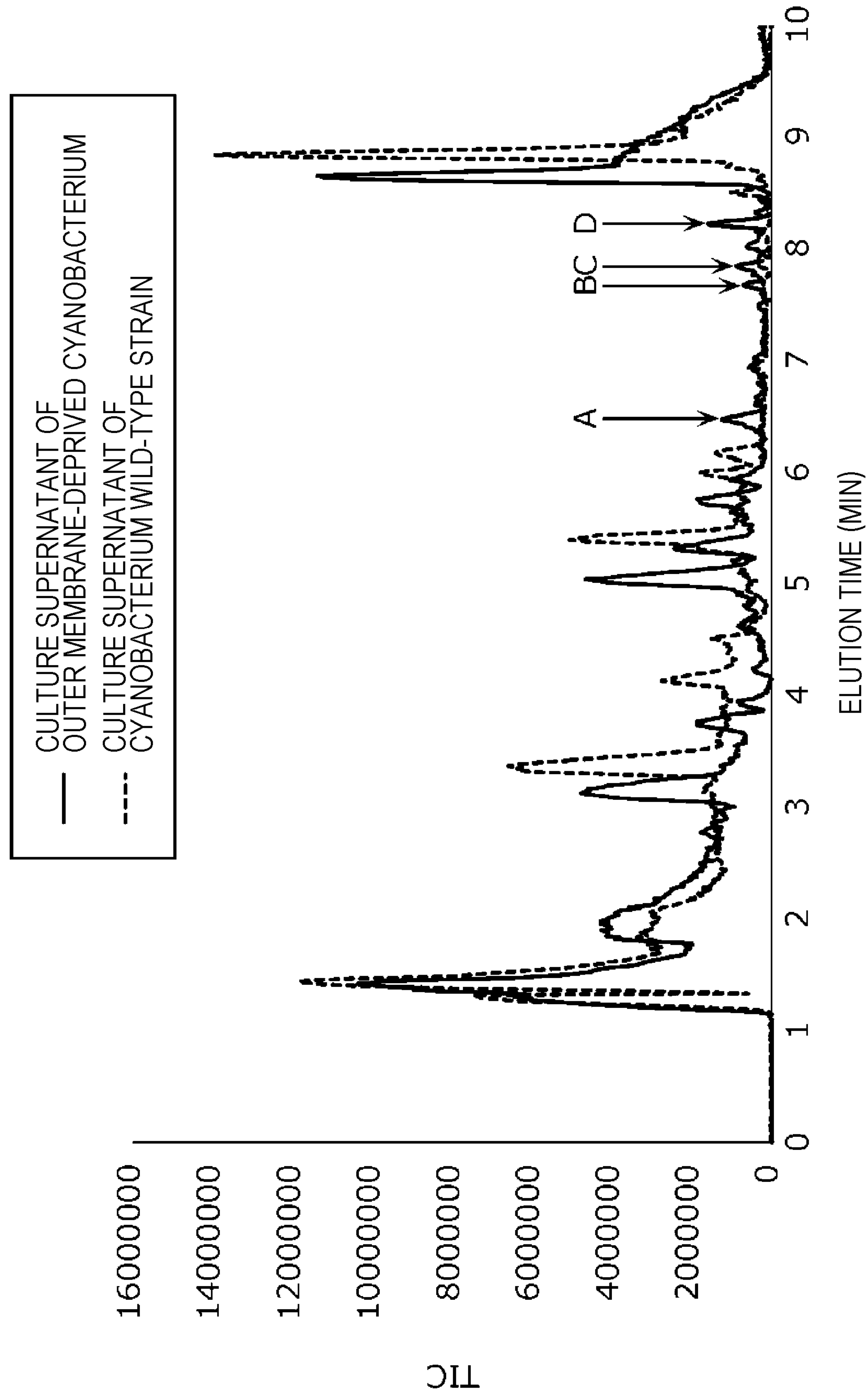
CULTURE SUPERNATANT OF OUTER MEMBRANE-DEPRIVED CYANOBACTERIUM
CULTURE SUPERNATANT OF CYANOBACTERIUM WILD-TYPE STRAIN
TIC
160000000
140000000
120000000
100000000
80000000
60000000
40000000
20000000
0
0
1
2
3
4
5
6
7
8
9
10
ELUTION TIME (MIN)
A
B
C
D

# FIG. 12

SEQ ID NO: 1 : Synechocystis sp. PCC 6803
MLKLSWKSLL VSPAVIGAAL VAGAASAAPD NVTNAQVLDQ LDQYTAEGQS SAIDQVTSVS 60
ELRDVQPTAW AYEALKSLVE RYGCIVGYPD RTFRGDRALS RWEFAAGLNA CMNVMERLIQ 120
ENVAVLREDI DKLKRLMQEF EAELAALGAR VDNLEARTSF LEDHQFSTTT KLTGEVIFAP 180
TAIFGTKKTL NNQTDNNQAV FQNRVRLQFN TSFSGEDLLV TRLAAGNGNR FKSFYPTQNV 240
IDPETGNPIG IVGDYYESPT FTQVHQLSPG DNNNVAVDWL AYYVPLDLGE NFRLNNYIAA 300
WGGIWDDFVP TLNPYFDDYT GGKGSLSQFT AQNPIYSIGG GTGIGTSLEL GFLSNLLGPT 360
SLSLGYLAST GNNPSSGGSV TNPATGNNYD FSSGGNGLFN GGYSALAQIT TNIFDRVSLG 420
FTYVNAYTTP DAAIFGKGGT QGIVGTTAAN LNRSELNDDF VNGLGVGAPP VDAATYNNNV 480
SGGSTSGNVI NPYDFGGKQT NSYGVQMAWN IADWLSFSAY GSYTNVTLIG KDNGDIWTYG 540
GGFAFPDLGK EGNVLGIFAG VQPYVSGFTN NTLGTTFVTT ANPLQVELFY KYQLTDNLSI 600
TPGVIWISKP EQTTNATDAF IGTVRGTFTF 630

SEQ ID NO: 2 : Synechococcus sp. NIES-970
MSKAMRNLLS GATVALGATV AFSGAAIAEE NQAVLDQISQ YSTDNSVAQV NSVFQLSDVS 60
PSDWAFDALR NLVENYNCIV GYPDGTFRGT RPLSRYEFAA GLNACLQQIE RMLEGGGEVT 120
QEDLAALRRL LNEFEAELAT LGARVDDLDG RVEFLEDNQF STTTKLNGEV IFNLGGAFGD 180
SRADGPGPID DEFTFSNRVR LNLDTSFTGR DRLRTRLEAG NITRLDRATG VDAARLGFDT 240
NNGNDVEIGK LFYRFPVGDN ITAYVGAVGL GLDDIFDVTN PYFESSGNGA LSRFGRFNPI 300
YRGPDGAGAG FNFDLGERVT FSAAYLADDG GASSPVSKNG LFNGNYTAGA QLDFAPSDNI 360
MLSAVYTRRY QQGGDVNLSG STGSDFAADP FGFAASSNNF GLQGSWLIGE KFNLGGWVGY 420
TNADLKDPVV TGNADVWNWA VNAALLDVGG EGNTLGFIFG QPPKLTEADI DFGGGPITFD 480
GSDSTYLAEL QYRYKVNNNI TITPGAYVLW NPNQNDANDT IVVGTVRTTF SF 532

SEQ ID NO: 3 : Anabaena cylindrica PCC 7122
MSNLLWKSLV VSPAVLGAAL LVSGTAIASP AAGNSASNAA TEVSILETQA ATEVVQQPVV 60
LAQVKQDTQV LDQVKRYSNE GQNLQSQVTS VSQFSDVQPT DWAFQALQSL VERYGCIAGY 120
PNGTYRGNRA LTRYEFAAGL NACLDRVNEL IATATADMVT KQDLATLQRL QEEFSAELAT 180
LRGRVDALEA RTAELEANQF STTTKLQGEA IFALTDAFNG DIAGKTVFQD RVRLNLQSSF 240
TGRDMLNTRL AAGNAQNFGN TGESRQTFDV GSTGTNNVVL DKLTYEAPVG PAQVYIAATG 300
GKHSHYAAVN NPYFFDKTDG GNGALSTFSS ENPIYRIGGG SGIALNVPFG QGGGILKPSS 360
LTLGYLASEA NSPAAGEGLF DGNYAALGQL NFNVGNRIAL AATYVHGYHG ASSRLFDSGE 420
GDDIVGTSLA NNLSLGSASS SNSYGVSAAF RPSDKLSVSG FVSYHDITGF GAGDDYEAWS 480
YGVGVALPDF GKKGNVLGIF GGAQPYSFNN NGGANSGDIP YQVEGFYKYR VSDNISVTPG 540
VIWLPSIGQS SSSEDTFIGT LRTTFTF 567

# FIG. 13

SEQ ID NO: 4 : Synechocystis sp. PCC 6803
MRVILCGYYG QDNAGDEALL VCLLQMLPAT VEPVVLSANP QVTTARYGVK AHYNRDWGKI 60
WQLLGQCDGF IWGGGSLMQD VTSVVSPLYY GGLMAIAQMR RLKTIAWAQG IGPLRRPPLR 120
WFTQQVLRGC SGISVRDQAS LQLVKQWGLK AFLAADPVWS LAAENASSSP SPLPMVAVNL 180
RAHPLLTLER LAVITEALRD FQHQTQTHVR LIPLQKSQDL AIAEAIAVEL PGSHEILYRP 240
DPRECKGLFR GAEFTIGMRL HSLIMAAAEG SACFALSYDP KVSRLIAEVG LPGRELADLP 300

SEQ ID NO: 5 : Synechococcus sp. PCC 7942
MRALLCGYYG EGNGGDEALL AALLQLLPPT VEPLVLSGNP AATRTCYGVE ACDRRSGAAV 60
WQALRRSDLF IFGGGSLIQD VTSWTSPLYY CGLMGLAQQL GLKTIAWAQG IGPLQRKRTR 120
WLARRTFQHC DRITVRDRGS AALLKEWGIS VAIAPDPVWA LEPLPLDQPL SNQEAIAVCL 180
RPHPELTPER SDRLKAALTT LQSQTQAPIL LIPFHHQQDR PLAAQWASEI PQAEVLDWQH 240
PRQLLSYFQS VRLTIAMRFH GLVMAAAAGN RCFGLSYDPK VRRFLDALEQ PGWDLPEIPD 300
SAEAIAAAWL ESWQAEPIHA AAIADLRQQV DVHRQALVF 339

SEQ ID NO: 6 : Anabaena cylindrica PCC 7122
MVKIRALLSG YYGKGNGGDE ALLATLLQML PPDVTPVVLS GNPEETKKRY GVESYNRMAF 60
LQIIKALRSC DAFIWGGGSL MQDATSSISP FYYGGLMALA QAMGLKTVAW GQGIGPLLRS 120
QTRWLAKRNF ALCTKISVRD RSSAALLSNW GIPHILAPDP VWALESKPVT ELADLPKPRI 180
AVILRNHPQL TETRLANLIH ALVDLQQATQ AFILLLPFQK SEDLDIAEKI QLQLKDVSKI 240
ICAEDPQLLK GIFRGVEMAI GMRLHSLIMA ASEGCRCFAL SYDPKVNHLM EDLEIPGWDL 300
KDLPTDVDLM SKTWIQYYHN SEALSSEKIQ SLVNGAFLHR DLLRASLSNK 350

# FIG. 14

SEQ ID NO: 7 : Synechocystis sp. PCC 6803

atgcttaaac tatcttggaa gtccctgtta gtgagccctg ctgtgatcgg tgccgcccctt 60
gtggctggtg ctgctagtgc tgctcccgac aacgttacga atgctcaagt tttagaccaa 120
ttagaccaat acactgccga aggtcagtct tctgccattg atcaagtaac cagcgttagc 180
gaactccgcg atgtgcaacc cactgcctgg gcctatgaag cgctcaagag cttggtggaa 240
cgctacggtt gtatcgttgg ttaccctgac cgtaccttcc gtggtgatcg agccctcagc 300
cgttgggaat ttgccgctgg tcttaacgcc tgtatgaacg ttatggaacg tttgattcag 360
gaaaacgtgg ctgttctccg ggaggacatc gacaaactga aacgcctcat gcaggaattc 420
gaggcagaat tggctgcttt gggtgctcgg gttgataacc tcgaagcccg cacctctttc 480
ctcgaagacc atcagttttc taccaccacc aaattgaccg gggaagtaat atttgctccc 540
accgccattt ttggtaccaa aaagactctc aacaaccaaa ccgataacaa ccaagcggta 600
ttccagaacc gggttcgttt acagttcaac accagcttct ccggtgaaga cctgttggtc 660
actcgtctcg ccgctggtaa cggtaatcgc ttcaagagct tctatcctac tcagaatgtt 720
attgatcccg agaccggtaa ccccatcggt attgtaggtg actactatga aagccccacc 780
tttacccagg tgcaccagct ttcccccggc gacaacaaca acgtagccgt tgactggtta 840
gcttactatg ttcccttgga tctgggcgaa aacttccgtc tcaacaacta cattgctgct 900
tggggcggta tctgggatga cttcgttcct accctgaacc cctatttcga cgactacact 960
ggcggcaaag gttccttgag tcagttcacc gcccagaacc ccatctactc cattggtggc 1020
ggtactggta taggtacaag cctagagttg ggcttcctca gcaatctgtt gggccccact 1080
tccttgagct tgggttactt ggctagcacg ggcaacaacc ccagcagtgg tggttctgtt 1140
actaaccctg ccactggcaa caattatgac ttcagcagcg gtggtaacgg tttgtttaac 1200
ggtggctata gcgccttggc tcaaattacc accaacattt tcgaccgggt tagcctaggc 1260
ttcacctacg ttaatgccta caccacccct gatgccgcta tcttcggtaa aggtggcact 1320
caaggtatcg tcggtaccac tgcggctaac ct 1352

# FIG. 15

SEQ ID NO: 8 : Synechococcus sp. NIES-970

atgtctaaag caatgagaaa tcttctcagt ggtgcaaccg ttgctctcgg tgcaactgtt 60
gctttttctg gcgcagcgat cgccgaagaa aaccaagccg tcctcgacca gatcagtcaa 120
tacagcactg ataactccgt tgctcaggtc aacagcgttt tccagctcag cgacgtttcc 180
ccttccgatt gggcttttga cgcgctccgc aacttggttg aaaactacaa ctgtatcgtt 240
ggttatcctg atggtacttt ccgtggcact cgccccctca gccgttatga gttcgccgca 300
ggtctgaatg cttgtttgca acagatcgaa cggatgctcg aaggtggcgg tgaagtaacc 360
caagaagatc tcgcagccct ccgtcgtctg ctcaacgaat ttgaagcaga actagctacc 420
ctcggcgctc gtgttgatga ccttgatggc cgtgttgagt tccttgaaga caatcagttc 480
tccaccacca ccaaactgaa tggggaagtt atcttcaacc ttggtggcgc ttttggcgat 540
agcagagctg atggtcctgg cccaatcgat gatgagttca cattcagcaa tcgtgttcgt 600
ctgaacctag acactagctt cactggtcgc gatcgcctcc gcactcgtct tgaagctggc 660
aatatcactc gcttagaccg tgcaactggt gtagatgcag cccgtctcgg ctttgacact 720
aacaacggta atgatgttga aattgggaaa ttgttctacc gcttccctgt aggtgacaac 780
attactgcct atgtcggtgc cgttggccta ggtcttgatg acatatttga tgtaaccaac 840
ccatattttg agagcagcgg taatggtgcc ctctctcgtt tcggtcgttt taacccccatc 900
taccgtggcc ccgatggtgc tggtgctggt tttaacttcg atcttggtga aagagttacc 960
ttctccgcag cttaccttgc ggatgatggt ggtgctagca gccctgtctc gaagaatggc 1020
ctgttcaatg gcaactacac tgcgggtgct cagctcgatt ttgctccctc tgacaacatc 1080
atgctgtctg ccgtttatac ccgtcgctac caacagggtg gcgatgtcaa cctgagcggt 1140
agcactggta gtgattttgc tgcagatcct tttggttttg ctgcttcttc caacaacttc 1200
ggcttacaag gtagctggtt gatcggtgaa aaattcaacc ttggtggctg ggtcggctac 1260
accaatgctg atctcaaaga tcctgttgtc accggtaacg ctgatgtttg gaactgggct 1320
gttaacgccg ctctacttga tgtcggcggc gaa 1353

# FIG. 16

SEQ ID NO: 9 : Anabaena cylindrica PCC 7122

atgtctaatc tattgtggaa atccttggtg gttagcccag ccgtgttggg agccgcctta 60
ttagtttcag gaacagcgat cgcatcgccc gccgcaggca actcagcctc aaatgctgct 120
actgaagtat caatactaga aacacaagcg gcaacagagg ttgttcaaca acctgtagta 180
ttggctcaag tcaagcaaga cactcaggtt ttagatcaag ttaaacgcta cagtaacgaa 240
ggtcaaaatc tacaatctca agtaacatca gtctctcagt tttccgatgt ccaaccaact 300
gactgggcat tccaagcttt gcagtccttg gtggaacgtt atggttgtat cgcaggttat 360
cccaatggta catatcgcgg aaaccgtgct ttaacccgtt atgaatttgc cgctggtttg 420
aatgcctgtt tagaccgggt gaatgaattg attgccacag caacagctga catggtgaca 480
aaacaggatt tagccacctt acagcgcttg caagaagaat tttctgctga attggcaact 540
ttacgcggtc gtgtcgatgc tttggaagca cgcactgctg agttagaagc taatcaattc 600
tctactacca ccaaattgca aggtgaagct atttttgctc tgaccgatgc ttttaatggt 660
gacattgccg gtaagaccgt ttttcaagat agagtgcgtt tgaacctgca aagcagcttc 720
acaggtcgag atatgttgaa tacccgtttg gctgcgggta atgcacaaaa ctttggaaac 780
actggggaaa gtagacaaac ctttgacgtt ggtagcactg ggactaacaa tgttgtgtta 840
gacaaactga cctatgaagc tcctgttggt ccagcacagg tctacatagc agctacaggt 900
ggtaaacaca gccattatgc tgctgttaac aacccttact tctttgataa aactgatggt 960
ggtaacggtg ctttaagtac ctttagttct gaaaacccca tctatcgaat tggtggtggt 1020
tctggtattg cccttaatgt acccttttggt caaggtggcg gtattctcaa accaagttca 1080
ttgacattgg gttatttggc ttctgaggct aatagtcctg ctgctggtga aggtttgttc 1140
gacggtaact atgctgcttt aggtcagtta aactttaacg ttggtaatcg cattgcctta 1200
gctgctactt atgtacatgg ttatcatggt gctagtagcc gtttgtttga ctcaggagag 1260
ggtgatgata tcgtaggtac atcactagct aataatctca gtttaggtag cgcctcgtcc 1320
agcaactcct acggtgtttc tgctgccttc agacctagcg 1360

# FIG. 17

SEQ ID NO: 10 : Synechocystis sp. PCC 6803

atgcgtgtca ttctgtgtgg ttactacggc caagacaatg ctggggatga agccctttta 60
gtctgtttgc tgcaaatgct tcctgcgacg gtggagccag tggtgctctc cgccaatccc 120
caggtcacca cagcgagata cggtgttaaa gcccactaca accgagactg ggcaaaattt 180
tggcagcttt tgggtcaatg tgacggtttt atctgggggg gaggcagttt gatgcaggat 240
gtgaccagcg tggttagtcc cctatactac ggtggtttga tggcgatcgc ccagatgcgg 300
cgtttaaaaa ccattgcctg ggcccagggc attggccccc ttagaaggcc gccgttgcgc 360
tggtttaccc aacaggtgct ccggggttgt agcggcatca gcgtacggga tcaagcttct 420
ttgcaattag ttaaacaatg gggcttaaag gctttttttag cggcggatcc ggtgtggtct 480
ttagccgctg aaaatgcgtc ttcttccccc agccctttgc ccatggtggc ggtgaatttg 540
cgggcccatc ctttgttaac cttggaacgc ttggcggtca ttaccgaagc cctaagggat 600
tttcaacacc agacccaaac ccatgtgcgg ctcattcccc tacaaaaatc ccaggatttg 660
gccattgccg aagcgatcgc cgtggaattg ccggggagtc atgaaattct ttaccgcccc 720
gaccccaggg aatgtaaagg tttgttccgg ggggcggaat tcaccattgg catgcggctc 780
cacagtttaa tcatggccgc ggcggagggc agtgcctgct ttgcccttag ttatgaccct 840
aaagttagtc gtttaatcgc agaggtggga ctaccgggcc gggaattggc ggatttaccc 900
agggatagca acgaactcag ccaggtttgg cagggacatt tccggcaaag acaaccctct 960
accggggtag agttcttgca aaaatctgcc cttcagcatc aaactttgtt gcagaaaatt 1020
tttgttgact ag 1032

# FIG. 18

SEQ ID NO: 11 : Synechococcus sp. PCC 7942

```
atgcgggcgt tgctctgtgg ctattacgga gagggcaatg ggggagatga ggcgctgttg 60
gcagcgctgt tgcaactctt gccgccgaca gtggaaccgc tggtgctgtc ggggaatcct 120
gcggcaactc gcacttgcta cggcgtagaa gcttgcgatc gccgatcggg ggctgcggtt 180
tggcaggcac tgcgacgcag tgatctattc atctttggcg gcggcagcct gattcaagat 240
gtcacaagct ggacgagccc tctctactac tgcggtctga tggggctggc acaacagtta 300
ggtctcaaaa cgatcgcctg ggcccagggc attgggccac tgcagcgcaa acgaacccgc 360
tggttggcac gccgaacgtt tcagcactgc gatcgcatta cagtgcgcga tcgcggttct 420
gctgccttgc taaaagagtg ggggatttct gtggcgattg ccccggatcc cgtttgggct 480
ttagagccgc tgcctttaga tcagccgctg tcgaaccaag aggcgatcgc ggtttgtctg 540
cggccccacc ctgagttgac gcctgagcgc agcgatcgct tgaaagctgc cctgacaact 600
ctgcaatcgc aaacccaagc acccatcctg ctgattccct tccaccacca gcaagatcga 660
ccactggccg cgcaatgggc aagcgaaatt ccccaagcag aagttttgga ctggcagcat 720
ccgcgccagc tcctcagcta ctttcagtcg gtgcgattaa cgatcgccat gcgcttccat 780
ggattagtga tggcagcagc ggctggaaat cgttgctttg gcctcagcta tgaccccaaa 840
gtgcgccgtt tcctggatgc ccttgagcaa cccggctggg atttgccgga gattccagac 900
tctgctgagg cgatcgcggc cgcttggtta gagtcttggc aagcagaacc gattcatgcc 960
gctgcgatcg cggacctccg tcagcaagtg gatgtccatc gccaggcttt agttttttga 1020
```

# FIG. 19

SEQ ID NO: 12 : Anabaena cylindrica PCC 7122
ctacttatta ctcaatgatg ctctcaataa atcccgatgt aaaaaagctc catttactaa 60
agattgtatt ttctcagatg ataatgcttc gctattgtga taatattgaa tccaggtttt 120
actcattaaa tctacatcag ttggtaaatc ctttaaatcc catccaggta tttctaaatc 180
ttccatcaaa tggttaacct tggggtcata actcaaagca aaacaacgac aaccttcact 240
agctgccata attaaactgt gtagacgcat ccctattgcc atttctacac cacgaaatat 300
acctttaaa agttgtggat cttctgcaca gataatttta ctaacatctt ttaattgcag 360
ttgaattttt tctgcaatat ctaaatcttc actttttga aaaggcaata ataaaataaa 420
tgcttgggta gcttgttgta aatcaactaa ggcatggatt aaatttgcta accgagtttc 480
tgtaagttgg ggatgatttc ttaaaataac ggcaattcta ggttttggta aatctgctaa 540
ttctgttact ggtttagatt ctaaagccca aactggatca ggtgcaagaa tatgaggtat 600
gcccaattt gataataaag ccgcactaga gcgatcgcgt acactgattt tagtacaaag 660
agcaaaattt cgctttgcta accaacgagt ttgcgatcgc aataaaggcc ctattccctg 720
tccccaagct acagttttca aacccatcgc ttgagctaaa gccattaatc cccataata 780
aaaagggcta atgctgctag tcgcatcctg cattaaactt ccaccacccc agatgaaagc 840
atcacaggaa cgcaaagctt tgataatctg caaaaacgcc atacggttgt aactttccac 900
accataacgc tttttagttt cctcaggatt cccagaaagc accacaggcg ttacatcagg 960
tggtagcatt tgcagaagtg tcgccaacaa agcttcatca ccaccattac ctttaccgta 1020
atacccagac aataacgccc gtatcttcac cat 1053

SEQ ID NO: 13 : Artificial Sequence / Synthesized Primer
cagtgaattc gagctcggta tatagcgttg cagtccctgg 40

SEQ ID NO: 14 : Artificial Sequence / Synthesized Primer
gattacgcca agcttgcatg accgcggtca cttcataacc 40

SEQ ID NO: 15 : Artificial Sequence / Synthesized Primer
cagtgaattc gagctcggta ataaccgttg tcccttttgt ttcatcg 47

SEQ ID NO: 16 : Artificial Sequence / Synthesized Primer
tgttagtgag ccctgctgtt agctcccagt atctctatca ctgat 45

SEQ ID NO: 17 : Artificial Sequence / Synthesized Primer
acagcagggc tcactaacag ttttagagct agaaatagca agttaaaata a 51

SEQ ID NO: 18 : Artificial Sequence / Synthesized Primer
gattacgcca agcttgcatg gggaacaagc tgaatctggg catc 44

FIG. 20

SEQ ID NO: 19 : Artificial Sequence / Synthesized Primer
ttttagtctg tttgctgcat agctcccagt atctctatca ctgat 45

SEQ ID NO: 20 : Artificial Sequence / Synthesized Primer
tgcagcaaac agactaaaag ttttagagct agaaatagca agttaaaata a 51

SEQ ID NO: 21 : Synechocystis sp. PCC 6803
acagcagggc tcactaaca 19

SEQ ID NO: 22 : Synechocystis sp. PCC 6803
tgcagcaaac agactaaaa 19

## INTERNATIONAL SEARCH REPORT

International application No.
**PCT/JP2023/024903**

**A. CLASSIFICATION OF SUBJECT MATTER**

***C12Q 1/02***(2006.01)i; ***C12M 1/00***(2006.01)i; ***C12M 1/34***(2006.01)i; ***C12N 1/12***(2006.01)i
FI: C12Q1/02; C12M1/00 A; C12M1/34 A; C12N1/12 Z ZNA

According to International Patent Classification (IPC) or to both national classification and IPC

**B. FIELDS SEARCHED**

Minimum documentation searched (classification system followed by classification symbols)

C12Q1/02; C12M1/00; C12M1/34; C12N1/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | KUSAMA, Shoko et al. Order-of-magnitude enhancement in photocurrent generation of Synechocystis sp. PCC 6803 by outer membrane deprivation. NATURE COMMUNICATIONS. 02 June 2022, 13:3067, pp. 1-12, doi.org/10.1038/s41467-022-30764-z<br>particularly, abstract | 1-7 |
| A | WO 2021/100640 A1 (PANASONIC INTELLECTUAL PROPERTY MANAGEMENT CO., LTD.) 27 May 2021 (2021-05-27)<br>entire text, all drawings | 1-7 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2023** | **12 September 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/024903**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

"In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/024903**

| Patent document cited in search report | Publication date (day/month/year) | Patent family member(s) | Publication date (day/month/year) |
|---|---|---|---|
| WO 2021/100640 A1 | 27 May 2021 | US 2022/0325312 A1<br>entire text, all drawings<br>EP 4063483 A1<br>CN 114651060 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021100640 A

### Non-patent literature cited in the description


- Rerouting Carbon Flux To Enhance Photosynthetic Productivity. **DANIEL C DUCAT et al.** Applied and Environmental Microbiology. American Society for Microbiology, April 2012, vol. 78, 2660-2668
- Direct photosynthetic recycling of carbon dioxide to isobutyraldehyde. **SHOTA ATSUMI et al.** Nature Biotechnology. Nature Publishing Group, November 2009, vol. 27, 1177-1180
- Fatty acid production in genetically modified cyanobacteria. **XINYAO LIU et al.** The Proceedings of the Natural Academy of Sciences (PNAS). National Academy of Sciences, April 2011, vol. 108, 6899-6904
- **ARNAV DESHPANDE et al.** Combining random mutagenesis and metabolic engineering for enhanced tryptophan production in Synechocystis sp. strain PCC 6803. *Applied Environmental Microbiology*, May 2020, vol. 86 (9), e02816-19
- Alga-Produced Cholera Toxin-Pfs25 Fusion Proteins as Oral Vaccines. **JAMES A. GREGORY et al.** Applied and Environmental Microbiology. American Society for Microbiology, June 2013, vol. 79, 3917-3925
- **HIKARU KOWATA**. Studies on molecular basis of cyanobacterial outer membrane function and its evolutionary relationship with primitive chloroplasts. *Doctoral dissertation*, 27 March 2018, http://hdl.handle.net/10097/00122689
- **SEIJI KOJIMA**. Clarification and application of membrane stabilizing mechanism and substance permeation mechanism derived from bacteria that act on the chloroplast surface membrane. *KAKENHI (Grants-in-Aid for Scientific Research)*, 23 April 2018, https://kaken.nii.ac.jp/grant/KAKENHI-PROJECT-18H02117
- **JURGENS** ; **WECKESSER**. *J. Bacteriol.*, 1986, vol. 168, 568-573
- **KOJIMA et al.** *Biosci. Biotech. Biochem.*, 2016, vol. 10, 1954-1959
- **KOWATA et al.** *J. Bacteriol.*, 2017, vol. 199, e00371-17
- **KOJIMA et al.** *J. Biol. Chem.*, 2016, vol. 291, 20198-20209
- **MESNAGE et al.** *EMBO J.*, 2000, vol. 19, 4473-4484
- **YAO et al.** *ACS Synth. Biol.*, 2016, vol. 5, 207-212